# EUROPEAN PATENT APPLICATION

(11) **EP 0 974 667 A1**
(43) Date of publication of application: **26.01.2000**
(21) Application number: 98202375.6
(22) Date of filing: 16.07.1998
(51) Int. Cl.: C12N 15/82, C12N 5/10, C07K 14/415, A01H 5/00

(54) **Process to collect metabolites from modified nectar by insects**

(71) Applicant: CENTRUM VOOR PLANTENVEREDELINGS- EN REPRODUKTIEONDERZOEK (CPRO-DLO), 6708 PB Wageningen (NL)
(72) Inventor: Creemers, Jantina, c/o CPRO-DLO, 6708 PB Wageningen (NL); Angenent, Gerrit Cornelis, CPRO-DLO, 6708 PB Wageningen (NL); Kater, Martin Maria, CPRO-DLO, 6708 PB Wageningen (NL)
(74) Representative: De Hoop, Eric

(57) **Abstract**

The invention relates to a recombinant double-stranded DNA molecule comprising an expression cassette comprising the following constituents:
i) a promoter functional in nectaries of plants,
ii) a DNA sequence encoding a protein which is fused to the promoter,
iii) a DNA sequence encoding a signal peptide that targets the recombinant protein to nectar, which is translationally fused to the DNA sequence encoding the recombinant protein, and optionally
iv) a signal sequence functional in plants for the transcription termination and polyadenylation of an RNA molecule.
The invention further relates to a process for producing a recombinant gene product from honey, comprising:
i) producing a transgenic plant by introducing in a plant cell a recombinant double-stranded DNA molecule, regenerating plants from the transgenic cell, and selecting modified plants exhibiting excretion of the recombinant gene product in nectar,
ii) allowing insects, preferably bees, to collect nectar from the transgenic plants and to process the nectar into honey, and
iii) isolating and purifying the gene product from the honey.

## Description

### Field of the invention

The present invention relates to isolated, purified DNA sequences which can act as promoters in eukaryotic cells. More specifically, the present invention is related to such DNA sequences which act as promoters to express genes in nectaries of plants. The present invention also relates to chimerical gene constructs comprising a structural or a synthetic gene under the control of a promoter that effects expression of said genes in nectaries. This invention also relates to a process for producing metabolites in honey by allowing insects, preferably bees, to collect and process nectar from plants that excrete said metabolites in nectar or other exudates. Further, this invention relates to plant cells, plants or derivatives therefrom, that express the said chimerical gene.

### Background of the invention

Nectaries are nectar secreting organs or tissues that can be located inside (floral) or outside (extrafloral) the flower. The main component of nectar is sugar, the variation between nectars of flowers from different species mainly being the concentration and ratio of glucose, fructose and sucrose (Baker and Baker, 1982). In addition, depending on the plant species, varying amounts of polysaccharides, lipids, organic acids, volatiles, minerals, phosphates, alkaloids, amino acids and proteins have been detected (Baker and Baker, 1982). Being a specialised sink organ, the nectaries are supplied with sucrose by phloem unloading (Davis et al., 1985, Hagitzer and Fahn, 1992).

The mechanisms of sugar accumulation and nectar secretion have been described for several plant species (Fahn et al., 1979). Sugar transport to the nectaries is achieved by active transport mechanisms and/or osmotic and chemical gradients. In the nectaries of many plants sucrose is converted to glucose and fructose, resulting in a hexose dominant nectar. Part of the hexoses are converted to starch, which is hydrolysed prior to anthesis and nectar secretion. Cell to cell transport of nectar in the nectary parenchyma tissue is mainly symplastic, as demonstrated by the presence of many plasmodesmata between these cells (Fahn et al., 1979). Nectar is secreted from secretory cells via the cell membrane (eccrine secretion) or via the Golgi and endoplasmatic reticulum vesicles (granulocrine secretion). Research on the molecular regulation of nectary development and nectary biochemistry has not been reported.

The main function of floral nectar is to reward pollinating insects. Insects collect nectar to meet their short-term energy requirements. Colony-living honeybees process large quantities of nectar into honey, which is stored in honeycombs of the beehive and is used as food supply during the winter period. Within the bee colony different classes of worker bees cooperate in the honey production process. Foraging bees collect pollen and nectar from the flowers and bring it to the hive. On returning to the hive they give most of it up to household bees. Pollen is used as a protein source, especially to feed the brood. Adult nurse and worker bees use little protein, their capacity to digest proteins being very low (Crailsheim et al., 1993). Honey processing takes place by repeated swallowing and bringing up of the nectar from the honey stomach. In the first process 15% of the water content is lost. This semi-processed nectar is temporarily stored in a honeycomb cell and taken out later for further processing. The final process includes filtering the honey to discard small particles like pollen grains. Sugar metabolising enzymes (invertase, amylase) are added and the honey is concentrated to an average water content of 20%. Most nectars and honeys only contain traces of protein (<0.2%). However, *Calluna vulgaris* (heather) honey can contain up to 1.8% protein, giving it thixotropic properties (Butler, 1962). It is known that bees add enzymes like invertase to nectar during the honey processing. Therefore, the probability that proteases are also added is very low. Protein digestion does not take place in the honey stomach but in the intestine of the honeybee. However, the ability of adult worker bees to digest proteins is very low, their main requirement being energy which they obtain from nectar. Until now, it was not established which proteins are present in heather honey and whether these originate from floral heather nectar or are added to honey by honeybees.

In the present invention it was established that heather honey contains two unique proteins that originate from floral nectar of heather. Based on these results a production system for proteins in nectar and honey was established.

It is an object of the present invention to show that recombinant proteins can be secreted in nectar of transgenic plants, that this nectar is collected by honeybees and that the bees process this nectar into honey that contains the unaltered protein in a concentrated form.

### Definitions

**Honey:** A substance that contains approximately 80% sugar and varying amounts of other components and that is produced by insects, preferably bees, that collect and process nectar from floral or extrafloral nectaries, from honeydew, other plant exudates or artificial sugar solutions.

**MADS box gene**: a gene coding for a transcription factor having a region of 56 amino acids which is homologous to a similar region in the Arabidopsis AGAMOUS protein and Antirrhinum DEFICIENS protein. This region is called the 'MAIDS box'. At least 50% of the amino acids in this region should be identical to the amino acid composition in the MADS boxes of AGAMOUS and/or DEFICIENS.

**Nectary****:** secretory organ or secretory tissue of plants, located in the flowers (floral nectaries) or outside the flower (extrafloral nectaries) that excrete nectar.

**Nectar**: sugar containing fluid that is secreted by nectaries. Nectar can also contain substances like minerals, amino acids, proteins, organic acids, volatiles, alkaloids etc.

**Recombinant protein**: the gene product of a recombinant DNA molecule.

**Recombinant DNA molecule**: A DNA molecule in which sequences which are not naturally contiguous have been placed next to each other by in vitro manipulations.

**Promoter**: The DNA region, usually upstream to the coding sequence of a gene, which binds RNA polymerase and directs the enzyme to the correct transcriptional start site.

### Summary of the invention

The production of recombinant proteins for pharmaceutical purposes is a growing market. Until now, mainly bacterial and yeast systems have been used for bulk production of proteins. Recently animal production systems have also been developed. With the availability of efficient transformation techniques for plants, procedures to use plants for the production of proteins are now in progress. In plants, the recombinant proteins are targeted to sink organs like tubers and seeds. A serious draw-back of these production methods is that the recombinant protein can only be obtained after extended, and therefore expensive, purification steps.

The present invention provides a method to produce metabolites, preferably recombinant proteins in honey, which is manufactured by insects, preferably honeybees, that collect floral nectar of transgenic plants. Harvesting of honey is very simple and purification of the protein is very straight forward and requires no advanced purification steps. To give an estimation of the protein yield in a crop like rapeseed, we suggest an average protein production of 2% in honey, as has been found in honey of heather. If one hectare of rapeseed yields 100-500 kilo honey in one season, a yield of 2 to 10 kilo protein can be obtained. In addition, the present invention provides a method to collect metabolites from honey that is derived from non-transgenic plants that secrete these metabolites in nectar. An example are secondary metabolites like acetylandromedol, a diterpine compound, that is excreted in nectar of *Rhododendron arboreum* and *Rhododendron barbatum* and of *Piptanthus nepalensis* (Martini et al., 1990).

This invention provides a gene from petunia, *NEC1*, that is highly expressed in the nectaries of petunia and weakly expressed in the stamens. It also provides another gene from petunia, *FBP15*, that encodes a MADS box protein and which is specifically expressed in the nectaries of petunia. Further, it provides the isolated DNA sequences of the promoters of the *NEC1* and the *FBP15* genes. Furthermore, this invention provides an isolated DNA sequence expressed in nectaries encoding a signal peptide that is translationally fused to a germin-like protein (Lane et al., 1993, Dumas et al., 1995), having the function to target the mature germin-like protein to nectar of heather (*Calluna vulgaris*). This invention gives proof that protein-containing sugar solution is collected by honeybees to produce honey that has a higher protein content than the sugar solution itself, the protein having undergone no qualitative alterations. This invention also proofs that a recombinant protein can be produced in nectar of transgenic plants and that this protein is present in honey produced by honeybees that collected this nectar.

Accordingly, this invention provides an isolated DNA sequence which encodes a protein indicated NEC1 and having the amino acid sequence given in SEQ ID NO:1 of the sequence listing hereafter or homologs of NEC1. A homolog of NEC1 is predominantly expressed in nectaries and/or has at least 60% homology with the amino acid sequence given in SEQ ID NO:1. Further this invention provides an isolated DNA sequence which encodes a protein indicated FBP15 and having the amino acid sequence given in SEQ ID NO:2 of the sequence listing hereafter or a homolog of FBP15. A homolog of FBP15 is specifically expressed in nectaries and belonging to the MADS box family. Furthermore, a homolog is also a gene sequence that has at least 80% homology within the MADS box region and a 60% overall homology with the amino acid sequence given in SEQ ID NO:2. Further this invention provides the characterisation and the isolation of a DNA sequence which encodes a signal peptide indicated "CVSP" (Calluna vulgaris signal peptide), wherein the information contained in the DNA sequence permits, upon translational fusion with a DNA sequence encoding a protein that is expressed in nectaries, targeting of the protein to nectar.

The DNA sequences of the invention can also be characterised in that they comprise the *NEC1* gene and the *FBR15* gene having the nucleotide sequences given in SEQ ID NO:4 and SEQ ID NO:5 respectively, or a functionally homologous gene or an essentially identical nucleotide sequence or part thereof or derivatives thereof which are derived from said sequences by insertion, deletion or substitution of one or more nucleotides, said derived nucleotide sequences being obtainable by hybridisation with the nucleotide sequences given in SEQ ID NO:4 and 5 respectively.

Furthermore, the DNA sequences of the invention can also be characterised in that they comprise signal sequence CVSP having the nucleotide sequence given in SEQ ID NO:6, or an essentially identical nucleotide sequence or part thereof or derivatives thereof which are derived from said sequences by insertion, deletion or substitution of one or more nucleotides.

Further, this invention provides an isolated DNA sequence from the promoter region upstream of a nectary-specific expressed sequence, which nectary-specific expressed sequence encodes a protein comprising the amino acid sequence given in SEQ ID NO:1, or a homologous protein. Furthermore, this invention provides an isolated DNA sequence from the promoter region upstream of a nectary-specific expressed sequence, which nectary-specific expressed sequence encodes a protein comprising the amino acid sequence given in SEQ ID NO:2, or a homologous protein.

In a further aspect, the invention provides a protein encoded by any of the above defined DNA sequences. Further, the invention provides processes of producing transgenic plants exhibiting excretion of recombinant proteins in nectar, the expression of the chimerical genes and the targeting of the recombinant proteins being under the control of promoter sequences and a signal sequence as described in this invention. Still further, the invention provides processes of producing transgenic plants that produce recombinant proteins in nectar, the expression of the chimerical genes being under the control of promoter regions upstream of other genes that are expressed in nectaries. Still further, the invention provides processes of producing transgenic plants that produce recombinant proteins in nectar, the expression of these proteins being under the control of any signal peptide that affects targeting of a protein in nectar.

Also, the invention provides recombinant double stranded DNA molecules comprising expression cassettes to be used in the above process. Further, the invention provides transgenic bacteria, transgenic plants producing recombinant proteins in nectar, and also plant cells, tissue culture, plant parts or progeny plants derived from said transgenic plants. Finally, the invention provides a process to produce recombinant gene products in honey, produced by bees that collect nectar from transgenic plants and process this nectar into honey.

### Brief description of the figures:

**Figure 1** shows a polyacrylamide gel with PCR products after Differential Display mRNA amplification. PCR reactions were performed with the oligo-dT primer T12MG in combination with 5 different random primers Ap11-AP15 on cDNA samples of pistils without nectaries (two independent samples), nectaries (two independent samples), leaves and a mixture of sepals (s), petals (p) and stamens (a). Bold arrow depicts the cloned fragment DD18.
**Figure 2** is the DNA sequence of the Differential Display RT-PCR clone DD18a. The primers prat 122 and prat 119 that were used for 5' RACE PCR reactions are underlined.
**Figure 3** is the DNA sequence of clone RC8, obtained by RACE PCR with gene specific primers prat 122 and prat 119 (Fig. 2) in combination with adapter primers. Primer prat 129 (underlined) is used in the next step together with primer prat 122 to amplify the coding region of the *NEC1* cDNA.
**Figure 4** is the full length sequence of *NEC1* cDNA. The translation start (ATG) and translation stop (TAA) are depicted bold.
**Figure 5** shows the expression of *NEC1* (A) and *FBP15* (B) in wild type petunia plants (line W115) as determined by Northern blot analysis. Blot A contains total RNA, while blot B is enriched for mRNA. The tissues are indicated as: 1= leaf, 2= sepal, 3= petal, 4= stamen, 5= pistil, 6= nectary. For blot A the HindIII/EcoRI fragment of pDD18a was used as a probe. For blot B the full length cDNA of *FBP15* was used as a probe.
**Figure 6** Expression of *NEC1* by in situ localisation of *NEC1* transcripts (A) and activity of the *NEC1* promoter in the nectaries (B) and the stamen (C) as shown by GUS expression driven by the *NEC1* promoter. The GUS assay used for the stamens was incubated overnight without modifications to prevent diffusion (example 8). The GUS assay for the nectaries was incubated for 5 hrs, using an assay mixture to prevent diffusion (example 8). For in situ localisation longitudinal sections of flowers of *Petunia hybrida* were hybridised with digoxigenin-labeled antisense *NEC1* RNA
**Figure 7** is the DNA sequence from the promoter region upstream of a sequence encoding the NEC1 protein. Underlined is the translation start of *NEC1* cDNA.
**Figure 8** depicts a schematic presentation of the T-DNA region between the borders of the binary vector pBNEP1, containing the *NEC1* promoter (Figure 7), the *GUS* reporter gene and the nos terminator in pBINPLUS. This vector was used to generate transgenic plants to study the expression of the *NEC1* promoter.
**Figure 9** shows the SDS-PAGE separation of proteins that are present in commercial honey samples from different flowers. M= marker, lane 1: wattle bark, lane 2: flower mixture, lane 3: heather, lane 4: clover, lane 5: rapeseed.
**Figure 10** shows the SDS-PAGE separation of proteins that are present in commercial honey samples of rapeseed (RH2x, RH10x) and heather (HH2x, HH10x) and of nectar samples of rapeseed (RN2x, RN10x) and heather (HN2x, HN10x). M= molecular weight marker. Two (2x) or ten (10x) fold dilutions were used.
**Figure 11** shows the SDS-PAGE separation of proteins present in dilutions of the sugar/BSA feeding solution (A) and of honey from bees that had collected the sugar/BSA solution (B). The dilutions of the sugar/BSA and honey/BSA solution was the same for both gels: 1= 15x, 2= 30x, 3= 60x, 4= 75x, 5= 75x, 6= 90x, 7= 105x, 8= 120x, 9= 135x. M= marker
**Figure 12** shows the sequence homology of the N-terminal protein sequence of CVH29, a unique protein present in heather honey and nectar, with a germin-like protein GER1 from a gene bank homology search (BLAST).
**Figure 13** shows the deduced DNA sequence of the N-terminal protein sequence of CVH29. The degenerated primers prat 176 and prat 177 are underlined (A). The DNA sequence of the PCR product obtained with prat 176 and prat 177 performed on genomic DNA of heather is shown in B. The gene-specific primers prat 207 and prat 206 used to perform 5'RACE PCR reactions on cDNA from heather flowers are underlined.
**Figure 14** shows the DNA sequence of four independent clones obtained by 5'RACE PCR with prat 207 and prat 206 on cDNA of heather flowers. The ATG translation start of the putative signal sequence is boxed. The end of the putative signal sequence and the start of the mature protein are indicated by arrows.
**Figure 15** is the sequence of the synthetically produced DNA molecule encoding the signal sequence CVSP (boxed) with linkers.
**Figure 16** is the schematic representation of the plasmid pCV1. Not all restriction sites are indicated.
**Figure 17** is the schematic representation of the plasmid pCV2. Not all restriction sites are indicated.
**Figure 18** is the schematic representation of the plasmid pCV3. Not all restriction sites are indicated.

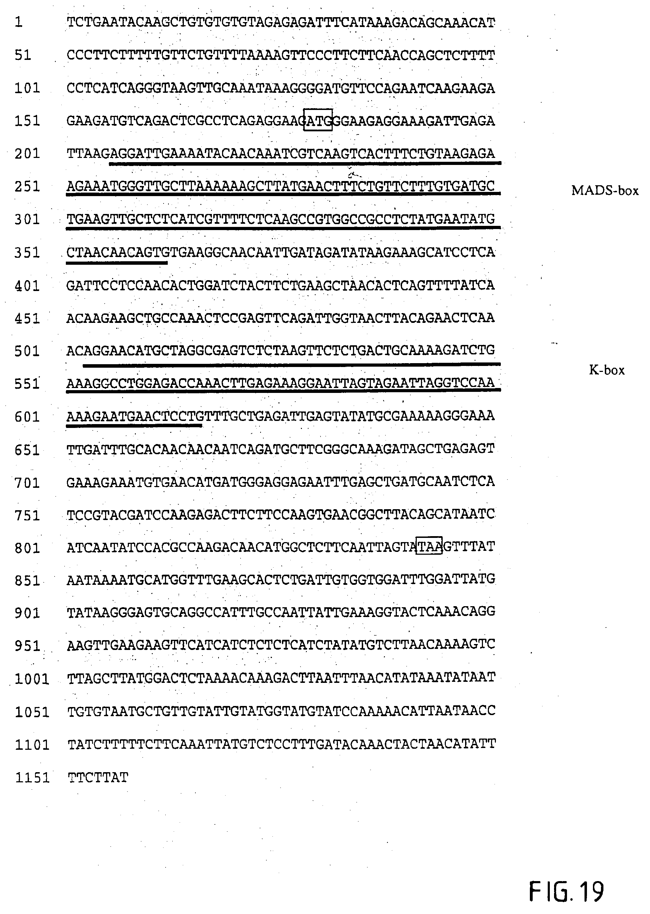

**Figure 19** is the DNA sequence of the full length cDNA of *FBP15*. The translation start (ATG) and translation stop (TAA) are boxed. The MAD-box and K-box region are underlined.

### Detailed description of the invention

This invention provides processes of producing transgenic plants that produce recombinant proteins in nectaries and nectar that is collected by foraging honeybees. This invention gives evidence that honeybees process protein containing nectar into honey that contains the unaltered protein in a concentrated form. Subsequently, the desired protein can be purified from the honey.

To express recombinant proteins in nectaries of transgenic plants, a translational fusion of an isolated DNA sequence from a promoter region upstream of a sequence encoding a protein that is expressed in nectaries with a sequence encoding the recombinant protein has to be carried out. Preferably, the isolated DNA sequence from a promoter region is upstream of a sequence that is specifically or highly expressed in nectaries.

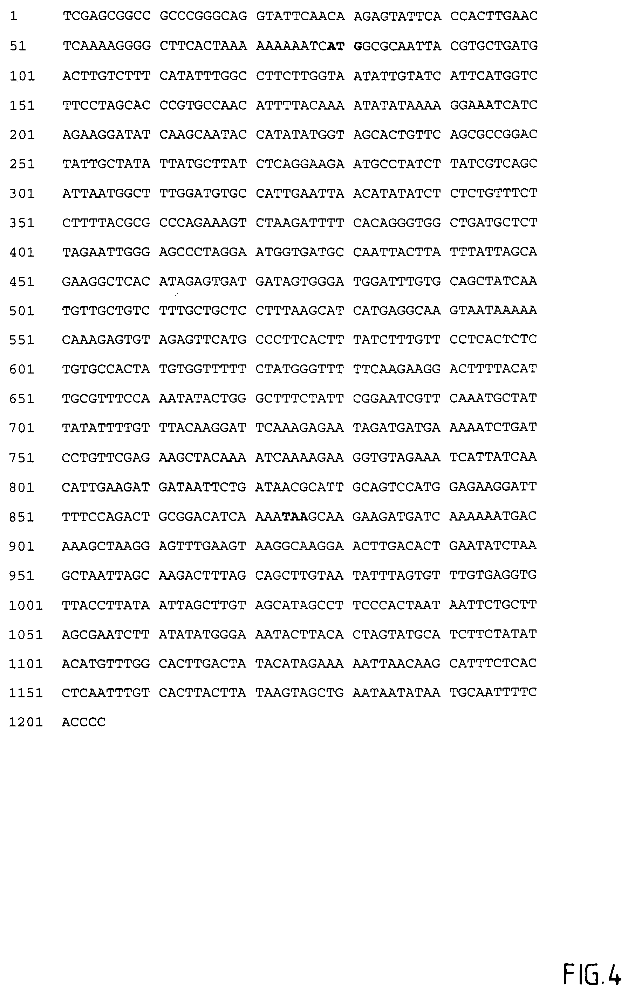

The invention relates to a DNA sequence isolated from *Petunia hybrida* that encodes a protein indicated NEC1 or a homologous protein or part thereof. A homologous protein has at least 65% homology with the amino acid sequence given in SEQ ID NO:1. The cDNA sequence of the *NEC1* gene is given in Fig. 4 and in SEQ ID NO:4. The deduced amino acid sequence of the *NEC1* gene is given in SEQ ID NO:1. The *NEC1* gene shows strong expression in the nectaries and in a very localised region of the anther filaments of *Petunia hybrida*. The deduced amino acid sequence of *NEC1* predicts a membrane bound protein. The precise function of the gene has not been elucidated yet, but considering the phenotype of transgenic plants that ectopically express *NEC1* in the leaves, a role in sugar metabolism of *NEC1* is apparent.

The present invention also relates to homologous DNA sequences that can be isolated from other organisms, preferably plants, using standard methods and the already known DNA sequence of the *NEC1* gene. More precisely, it is also possible to use DNA sequences which have a high degree of homology to the DNA sequence of the *NEC1* gene, but which are not completely identical, in the process according to the invention. The use of sequences having homologies between 85 and 100 % is to be preferred. DNA sequences can also be used which result from the sequence shown in SEQ ID NO:4 by insertion, deletion or substitution of one or more nucleotides. This includes naturally occurring variations or variations introduced through targeted mutagenesis or recombination. The DNA sequence shown in SEQ ID NO:4 can also be produced by using DNA synthesis techniques.

The invention also relates to a DNA sequence isolated from *Petunia hybrida* that encodes a MADS box protein indicated FBP15 or a homologous protein or part thereof. The cDNA sequence of *FBP15* is given in SEQ ID NO:5. *FBP15* shows exclusively expression in the nectaries of *Petunia hybrida*. The function of *FBP15* is unknown.
The present invention also relates to homologous DNA sequences that can be isolated from other organisms, preferably plants, using standard methods and the already known DNA sequence of *FBP15*. More precisely, it is also possible to use DNA sequences which have a high degree of homology to the DNA sequence of *FBP15*, but which are not completely identical, in the process according to the invention. The use of sequences having homologies between 85 and 100 % is to be preferred. DNA sequences can also be used which result from the sequence shown in SEQ ID NO:5 by insertion, deletion or substitution of one or more nucleotides. This includes naturally occurring variations or variations introduced through targeted mutagenesis or recombination. The DNA sequence shown in SEQ ID NO:5 can also be produced by using current DNA synthesis techniques.

Further, this invention provides an isolated DNA sequence from the promoter region upstream of a nectary-specific expressed sequence, which nectary-specific expressed sequence encodes a protein comprising the amino acid sequence given in SEQ ID NO:1, or a homologous protein that is expressed in nectaries. Furthermore, this invention provides an isolated DNA sequence from the promoter region upstream of an isolated DNA sequence from the promoter region upstream of a nectary-specific expressed sequence, which nectary specific sequence encodes a protein comprising the amino acid sequence given in SEQ ID NO:2, or a homologous protein that is expressed in nectaries.

More specifically this invention provides an isolated DNA sequence from the promoter region upstream of a nectary-specific expressed sequence, which nectary-specific expressed sequence has:
a) a nucleotide sequence given in SEQ ID NO:4, or
b) a nucleotide sequence obtainable by hybridisation with the nucleotide sequence of (a) or with a fragment of (a).

In a more specific embodiment this invention provides an isolated DNA sequence from the promoter region upstream of a nectary-specific expressed sequence, obtained from a plant of *Petunia hybrida*, the sequence consisting essentially of the sequence given in SEQ ID NO:7, or a functional fragment thereof having promoter activity.

In a further aspect, the invention provides an isolated DNA sequence from the promoter region upstream of a nectary-specific expressed sequence, which nectary-specific expressed sequence has:
a) a nucleotide sequence given in SEQ ID NO:5, or
b) a nucleotide sequence obtainable by hybridisation with the nucleotide sequence of (a) or with a fragment of (a).

In a more specific embodiment this invention provides an isolated DNA sequence from the promoter region upstream of a nectary-specific expressed sequence, obtained from a plant of *Petunia hybrida*, the sequence consisting essentially of the sequence given in SEQ ID NO:8 , or a functional fragment thereof having promoter activity.

Further, this invention provides an isolated DNA sequence comprising the coding region for a signal peptide, wherein the information contained in the DNA sequence permits, upon translational fusion with a DNA sequence encoding a protein that is expressed in nectaries, targeting of the protein to nectar. More specifically, the DNA sequence comprises the nucleotide sequence given in SEQ ID NO:6 obtained from a plant of *Calluna vulgaris*, or a nucleotide sequence obtainable by hybridisation with the nucleotide sequence given in SEQ ID NO:6. The use of sequences having homologies between 95 and 100 % is to be preferred. DNA sequences can also be used which result from the sequence shown in SEQ ID NO:6 by insertion, deletion or substitution of one or more nucleotides. This includes naturally occurring variations or variations introduced through targeted mutagenesis or recombination. The DNA sequence shown in SEQ ID NO:6 can also be produced by using DNA synthesis techniques. The signal peptide CVSP was isolated from nectar of *Calluna vulgaris* flowers and from honey processed by honeybees that collected the nectar. The function of CVSP in heather nectaries is to target the germin-like protein to nectar. The DNA sequence CVSP can also be used to target other proteins to nectar in plant species.

A subject of the present invention is the use of DNA sequences for producing recombinant proteins in nectar of plants, wherein the protein is produced in nectaries and targeted to nectar, and wherein expression in nectaries is achieved by using a DNA sequence consisting of the promoter region upstream of a DNA sequence that is expressed in nectaries, and wherein secretion in nectar is achieved by using a DNA sequence that encodes a signal sequence that targets the recombinant protein to nectar. In a further aspect the present invention relates to processes wherein a recombinant protein is expressed in other plant tissues than the nectaries and wherein the biochemical composition of nectar is changed as a consequence of the recombinant gene expression. The present invention also relates to processes wherein a recombinant protein is expressed in nectaries of a transgenic plant, wherein the biochemical composition of nectar or the nectar secretion is changed as a consequence of this protein expression. In particular, it relates to processes where the recombinant protein is an enzyme that interferes with the sugar metabolism in nectaries.

The production of a recombinant protein in nectaries and nectar is achieved by integrating into the genome of the plants a recombinant double-stranded DNA molecule comprising an expression cassette having the following constituents and expressing it:
i) a promoter functional in nectaries of plants,
ii) a DNA sequence encoding a protein which is fused to the promoter,
iii) a DNA sequence encoding a signal peptide that targets the recombinant protein to nectar, which is translationally fused to the DNA sequence encoding the recombinant protein, and optionally
iv) a signal sequence functional in plants for the transcription termination and polyadenylation of an RNA molecule.

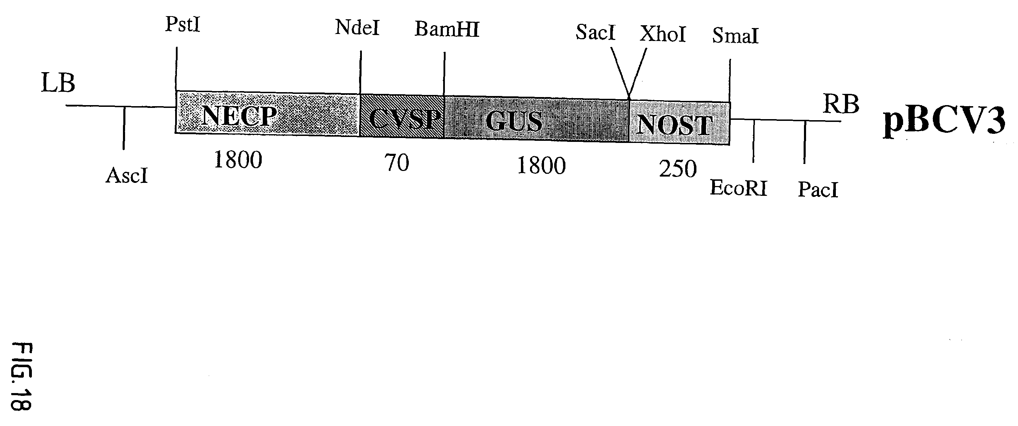

Such DNA molecules are also subject of the invention. The present invention provides an example of such a DNA molecule that contains the described expression cassettes in the form of plasmid pCV3 (Fig. 18), which comprises the promoter region of the *NEC1* gene from petunia, the signal sequence CVSP from heather, the coding region of the reporter gene *GUS* and the NOS terminator. In principle, any promoter that is active in the nectaries of plants can be used as promoter. The promoter is to ensure that the chosen gene is expressed in nectaries. Also, in principle, any signal sequence that targets the expressed protein to nectar can be used as a signal sequence. The signal sequence is to ensure that the protein is excreted in nectar. Furthermore, any sequence that encodes a recombinant protein in nectaries can be used in the present invention. Preferably, the subject of this invention relates to DNA sequences that encode proteins to be used for pharmaceutical purposes. It is also possible to use the invention to produce proteins for other purposes, e.g. enzymes for biotests or antioxidants for food additives. Furthermore, it is possible to use the invention to produce metabolites in nectar that attract predators of pest insects or that kill or repel pest insects. In another aspect it is possible to use the invention to produce metabolites in nectar that modify the attractiveness of the plant for pollinating insects or improve the health of pollinating insects.
It is also possible to use DNA sequences that encode proteins that modify the nectar composition or the sink strength of nectaries. This means that the recombinant protein interferes with metabolic pathways in the nectaries, resulting in changed levels of compounds that are already present in nectar, or the formation of new compounds in nectar.
In addition, the present invention also relates to expression cassettes that contain the above mentioned DNA sequences, except for a signal sequence. The recombinant protein is then only expressed in the nectaries, but not targeted to the nectar. Consequently, the expression of the recombinant protein in the nectaries can still affect nectar composition.
In a further aspect, the present invention also relates to expression cassettes that contain DNA sequences coding for a protein that is expressed in other tissues than the nectaries. The expression of the recombinant protein affects changes in the biochemical composition of nectar or in nectar secretion.
Finally, the present invention also relates to non-transgenic plants that produce metabolites in nectar that can be harvested and purified from honey that is produced by honeybees that collect this nectar. Examples for these metabolites are alkaloids, terpines, amino acids, proteins, pigments and volatiles.

A preferred embodiment of the process discussed above provides that the expression cassette is transformed to a plant species that produces nectar. Preferably, the recombinant protein is produced in nectar of plants that are visited by honeybees that collect the nectar. Honeybees collect floral as well as extrafloral nectar. The present invention relates to plants that produce recombinant proteins in floral or extrafloral nectar. In addition, the present invention also relates to plants that produce recombinant proteins in other plant organs, said plant organs producing an exudate that is collected by insects, preferably bees, and processed into honey. A particularly preferred embodiment of the present invention are plants that can be grown under controlled conditions. Controlled conditions are greenhouses or field facilities where transgenic plants can be grown according to the safety rules that are required. Preferably, the controlled conditions are such that bee colonies that perform normal foraging behaviour can be maintained in the same compartment during the flowering period. Preferred plants originate from the *Brassicaceae* family, in particular *Brassica napus*.

### Examples

### Example 1:

### Cloning of NEC1

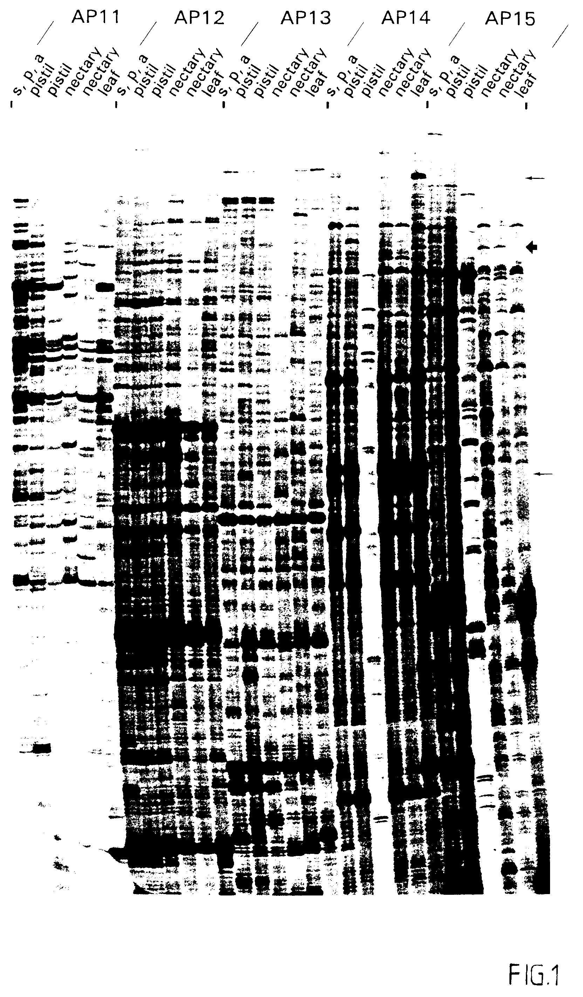

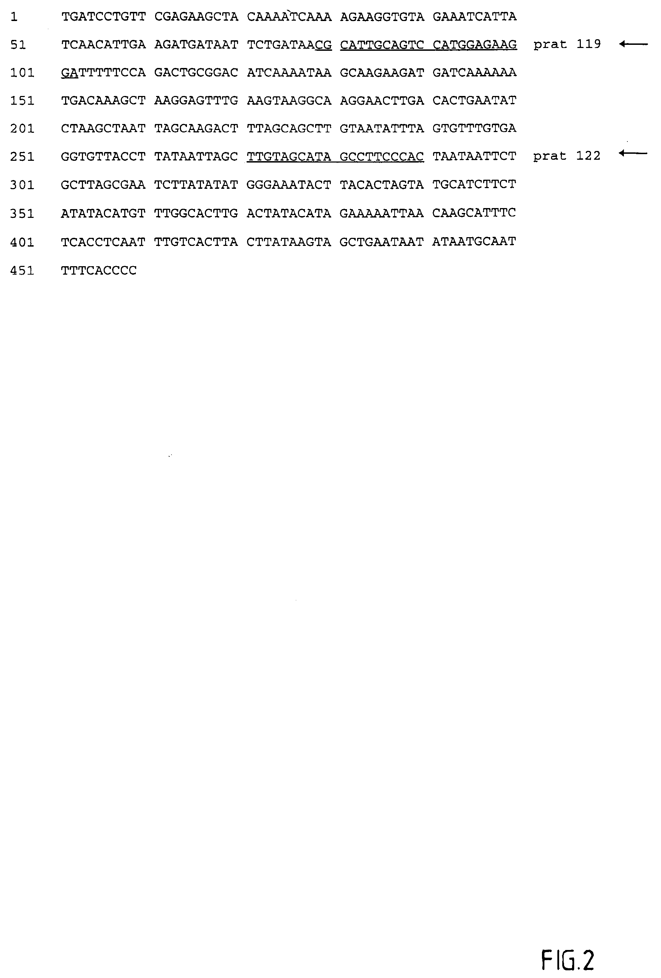

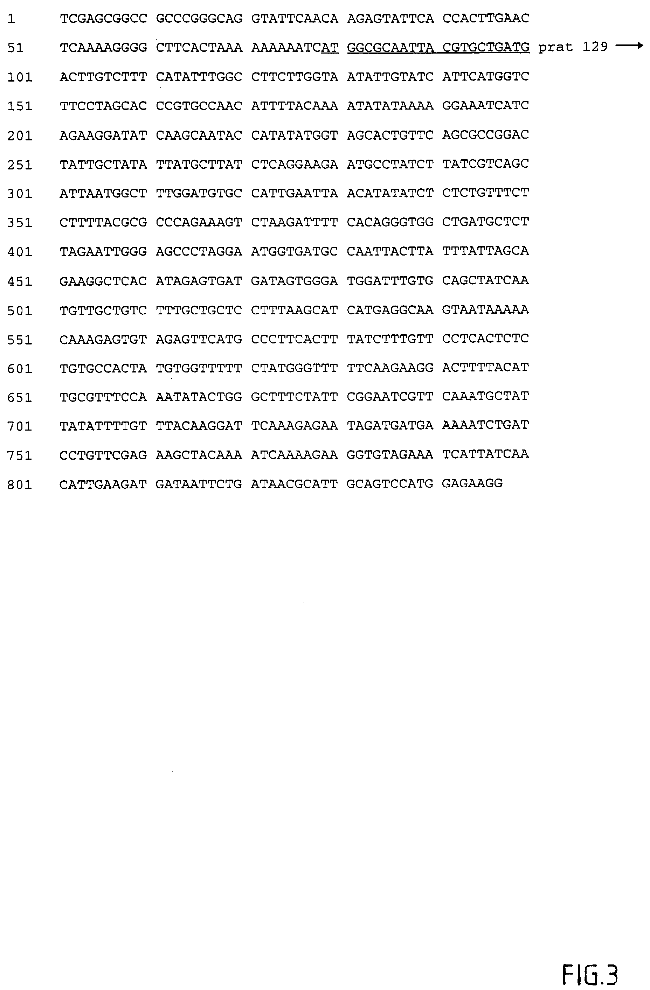

The *NEC1* cDNA was isolated using the mRNA Differential Display system (Genhunter Corporation, Brookline USA). The isolation of total RNA from nectaries, sepals, petals, stamens and pistils from open flowers and from young leaves of *Petunia hybrida* was done according to Verwoerd et al. (1989). Two independent RNA isolations were performed on nectaries as well as on pistils. A DNase treatment was carried out on each RNA sample, using the RNA MessageClean™ Kit (Genhunter Corporation Brookline USA, cat. No. M601). A reverse transcription reaction was carried out on 0.1 µg RNA of each sample, using the oligo-dT primer T12MG from the Genhunter Kit. Following the protocol, PCR reactions were carried out using the arbritary primers AP11-AP15 in combination with primer T12MG from the Kit. The PCR products were loaded on a sequencing gel and after electrophoresis the gel was blotted on 3M paper, dried and exposed to X-ray film (Figure 1). Two adjacent nectary-specific bands were cut out from the blot and the DNA was purified according to the manual. Reamplification of the fragment was carried out using the oligo-dT primer T12MG and the arbritary primer AP15. After electrophoresis, the PCR product was extracted from the agarose gel by freezing the isolated fragment in liquid nitrogen, followed by centrifugation. DNA was precipitated by adding 1/10 volume 1% HAc, 0.1M MgCl₂ and 2.5 volume of 96% ethanol to the supernatant. The pellet was dissolved in 10 µl TE buffer. The fragment, now called DD18a, was cloned into a PMOSBlue T-vector (RPN 1719, Amersham Little Chalfont UK) giving the vector pDD18a.
The nucleotide sequence of this 3' cDNA clone was determined by the dideoxynucleotide chain termination method (ABI PRISM™ Ready Reaction DyeDeoxy™ Terminator Cycle Sequencing Kit, P/N 402078, Perkin Elmer) and is shown in Figure 2. The DNA fragment has a length of 460 nucleotides. The missing 5' part of the cDNA was isolated using the Marathon TM cDNA Amplification Kit of Clontech (catalog K1802-1) and following the procedure as described in the manual. Briefly, Poly A+ RNA was isolated from nectaries of *Petunia hybrida* flowers. After double stranded cDNA synthesis, adapters were ligated and a 5'RACE reaction was carried out using the adapter primer AP1 supplied in the kit and a gene-specific primer prat 122. The nucleotide sequence of prat 122 is: 5'-gtgggaaggctatgctacaagc-3' (Figure 2). The PCR product was diluted 10x and 1 µl was used in a second 5' RACE reaction with the nested adapter primer supplied by the kit (AP2) and the nested gene-specific primer prat 119 (Figure 2). The nucleotide sequence of prat 119 is: 5'-ccttctccatggactgcaatgcg-'3 . After gel electrophoreses a fragment of ±850 bp was obtained that hybridised with clone DD18a. The fragment, now called RC8, was extracted from the gel, purified and cloned into a PMOSBlue T-vector as described above. The sequence is shown in Figure 3. The combined (overlapping) sequences of clones DD18a and RC8 are shown in Figure 4, comprising the full length cDNA of a gene called *NEC1* hereafter. The *NEC1* clone has a length of 1205 nucleotides and encodes for a polypeptide of 265 amino acid residues. Based on the deduced amino acid sequence, high homology was found with a cDNA that is associated with *Rhizobium*-induced nodule development in the legume *Medicago trunculata* (MtN3, gene bank number: gn1/PID/e274341). The percentages of identity and similarity are 47% and 72% respectively. Analysis of the predicted protein, using the CAOS/CAMM programme (Protein analysis 1991, Genetics Computer Group inc., Wisconsin USA), shows that the putative protein structure resembles membrane proteins, having six evenly spaced hydrophobic loops that traverse the cell membrane. In addition, a signal sequence is predicted at the N-terminus, while the C-terminus is highly hydrophilic. Highest homology with MtN3 is found in the N-terminal signal sequence, the first two membrane-spanning loops and the last two membrane-spanning loops. The C-terminal hydrophilic part shows the lowest homology (28% identity, 30% similarity). The function of *NEC1* has not yet been determined.

### Example 2:

### Cloning of FBP15

Petunia MADS box cDNA clones were isolated from a cDNA library made from nectaries of *Petunia hybrida* flowers. The cDNA library was constructed using the lambda ZAP cloning vector (Stratagene, La Jolla USA, catalog nr. 200400-200402). The library was screened under low stringency hybridisation conditions with a mixed probe comprising the MADS box regions of Floral binding protein gene *FBP2*, *FBP6* and *pMADS3* (Angenent et al., 1993, 1994, Tsuchimoto 1993). The hybridizing phage plaques were purified using standard techniques. Using the in vivo excision method, E.coli clones which contain a double-stranded Bluescript SK-plasmid with the cDNA insertion between the EcoRI and XhoI cleavage site of he polylinker were generated. Cross-hybridisation of the purified clones revealed 3 independent clones that did not cross hybridise with previously isolated FBP cDNA's and which were designated *FBP15*, *FBP16* and *FBP17*. The nucleotide sequence of *FBP15* was determined by the dideoxynucleotide-mediated chain termination method and is depicted in SEQ ID NO:5. The *FBP15* cDNA clone has a length of 1157 nucleotides and encodes a peptide of 222 amino acid residues. All characteristics of a MADS box protein are present in FBP15: a N-terminal located MADS box region which shows a high degree of similarity with other MADS box proteins, and a K-box in the middle of the protein with an alpha helical structure. *FBP15* is most similar to the tobacco MADS box protein NAG1, which is an Agamous homolog and expressed in whorl 3 and 4 (Huang et al., 1996, Mizukami et al., 1996).

### Example 3:

### Expression of FBP15

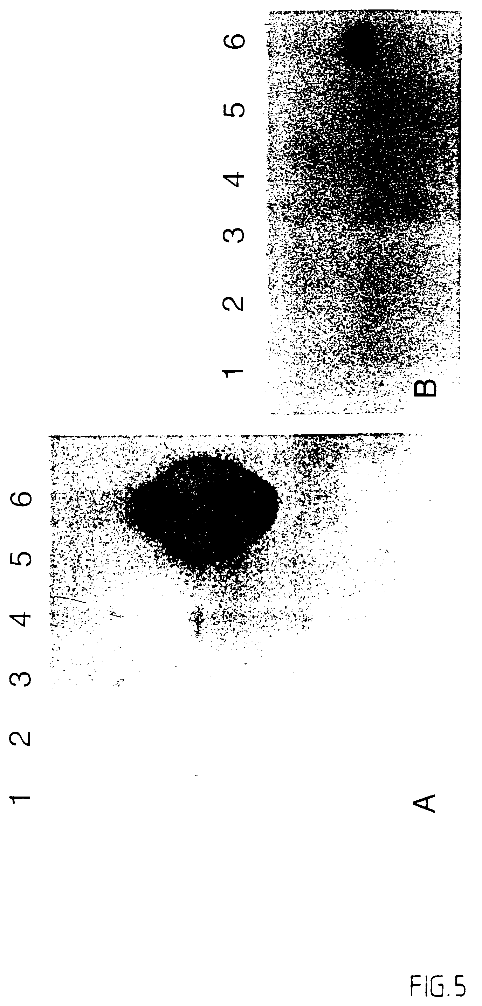

Expression of *FBP15* was determined by standard Northern blot hybridisation experiments. A DNA fragment comprising the complete cDNA of *FBP15* was used as a probe. High stringency hybridisation and washing conditions were used. Using 10 µg of total RNA from various petunia tissues, expression of *FBP15* was only detectable in nectaries. Using 10 µg of mRNA from various tissues, prepared by using the kit and protocol of the Quickprep Micro mRNA Purification Kit (Pharmacia Biotech), expression of *FBP15* was only detectable in nectaries as shown in Figure 5B.
The expression in the ovary and nectaries was determined by in situ hybridisation using a DIG labelled antisense RNA probe corresponding to the full length cDNA of *FBP15*. In vitro antisense RNA transcripts were made using T7 RNA polymerase. A standard protocol for in situ hybridisation was used as described by Canãs et al., 1994. A hybridizing signal was observed evenly strong in all cells of the nectary tissue.

### Example 4:

### Expression of NEC1

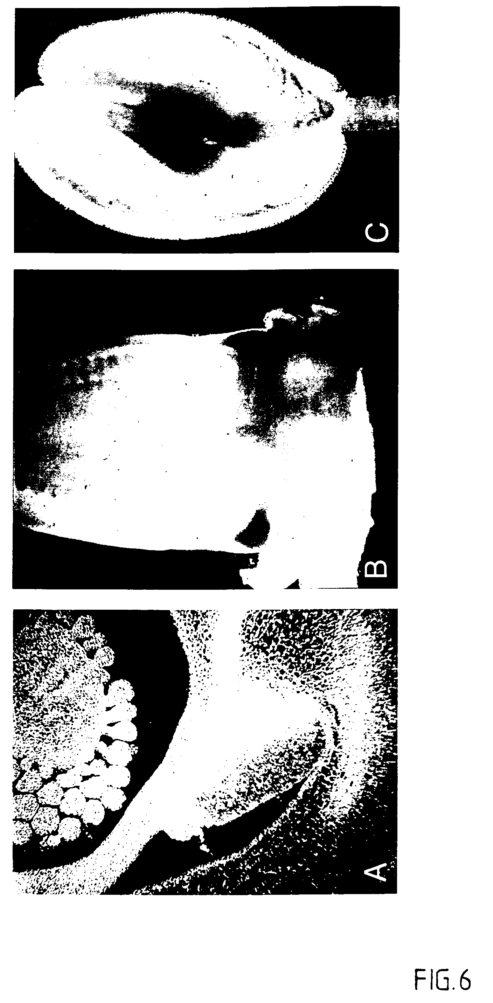

The RNA expression of *NEC1* was determined by standard Northern blot hybridisation experiments. A DNA fragment comprising the complete sequence of the Differential Display clone DD18 (Figure 2) was used as a probe. Using 10 µg of total RNA from various petunia tissues, strong expression of *NEC1* was detectable in nectaries and weak expression in anthers. No expression was detectable in other floral organs, in leaves or in roots (Figure 5A).
The expression in the ovary and nectaries was determined by in situ hybridisation using a DIG labelled antisense RNA probe corresponding to the nucleotides 79 to 1036 of *NEC1* cDNA, comprising the coding region and part of the 3' untranslated region. A clone containing this sequence was obtained by PCR on adapter-ligated cDNA, using two gene-specific primers prat 122 and prat 129 (Figure 4). The nucleotide sequence of prat 122 is: 5'-gtgggaaggctatgctacaagc-3' '', comprising the nucleotides 1015 to 1036 of the *NEC1* cDNA. The nucleotide sequence of prat 129 is: 5'-gggatccatggcgcaattacgtgctgatg-3', comprising the nucleotides 79 to 100 of the *NEC1* cDNA. The gene-specific region of the primers is underlined. The primer contains an extra BamHI and NcoI site at the 5'end. A PCR fragment of 958 nucleotides was obtained and cloned into a PMOSBlue vector. The fragment was subcloned in a vector containing the T7 promoter and in vitro antisense RNA transcripts were made using T7 RNA polymerase. A standard protocol for in situ hybridisation was used as described by Canãs et al., 1994. Strong hybridizing signals were observed in the outer cell layers of the nectaries (Figure 6A)

### Example 5:

### Isolation of NEC1 promoter fragment

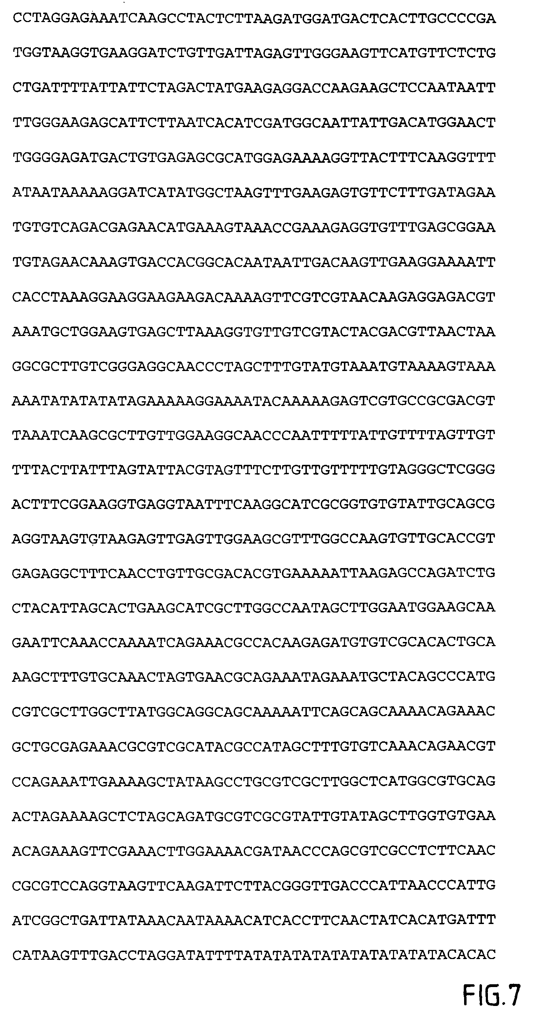

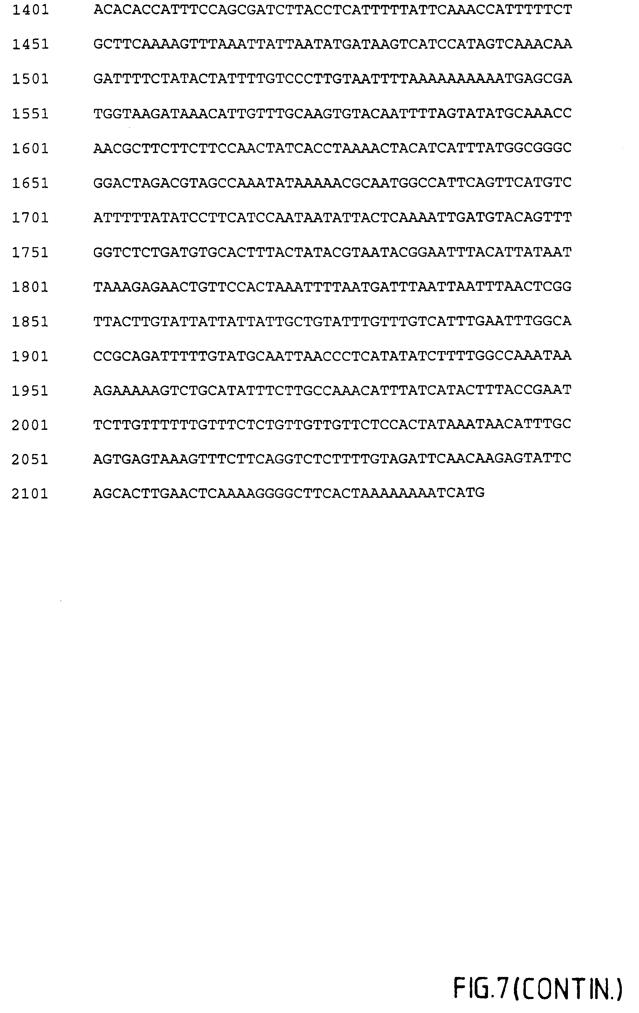

The promoter fragment of *NEC1* was cloned using the genome walker protocol (PT3042-1) and kit as provided by Clontech Laboraties. Briefly, genomic DNA from *Petunia hybrida* was digested with 5 different blunt cutting restriction enzymes. GenomeWalker adapters were ligated and PCR reactions were carried out on each Genomewalker "library" with a gene specific, reversed primer prat 148 and the adapter primer from the kit (AP1). The nucleotide sequence of prat 148 is: 5'-ccaagaaggccaaatatgaaagac-3' comprising the nucleotides 105 to 128 of the *NEC1* cDNA (Figure 4). PCR products were subjected to a second round of PCR, using the nested adapter primer AP2 and the nested gene specific, reversed primer prat 149. The nucleotide sequence of prat 149 is: 5'-aagtcatcagcacgtaattgcgcc-3', comprising the nucleotides 81 to 104 of the *NEC1* cDNA. From the second PCR a 2 kb fragment was isolated from the *StuI* library, which was cloned in the PMOSBlue T-vector, yielding the construct pMA5-10. Figure 7 (SEQ ID NO:7) shows the DNA sequence of the *NEC1* promoter in the construct pMA5-10, including the translation start of *NEC1* cDNA.

### Example 6:

### Construction of NEC1 promoter-GUS

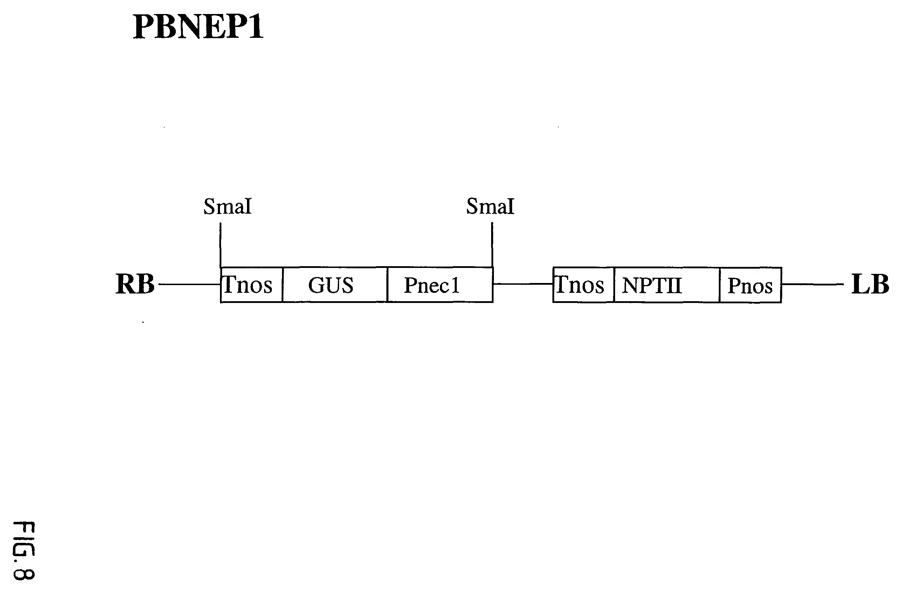

A PCR reaction was performed on pMA5-10 (example 5), using the forward vector primer U19 of pMOSBlue and the gene-specific primer prat 169. The nucleotide sequence of prat 169 is:
5'-cgctgcagcgccatggttttttttagtgaagcccc-3'. The gene-specific region is underlined. The primer contains an NcoI and BglII restriction site at the 3' end. The PCR product was digested with KpnI and NcoI and ligated into a pBluescript-derived vector (pMO4) that contains the NTM19 promoter (Custers et al., 1997), the reporter gene *GUS* and the nos terminator. The KpnI/NcoI NTM19 promoter fragment was replaced, resulting in a *NEC1*-promoter*/GUS* translational fusion. The resulting plasmid pNEP1 was digested with SmaI to release the *NEC1* promoter/*GUS*/nos fragment and this fragment was ligated into a derivative of the binary plasmid pBIN (Bevan, 1984) yielding the binary plasmid pBNEP1 (Figure 8). pBNEP1 was introduced into *Agrobacterium tumefaciens* strain LBA4404 or C58pMP90 by electroporation. Plasmid DNA from the Agrobacterium transformants was isolated and the structure of the binary vector was verified by restriction analysis and PCR.

### Example 7:

### Generation of transgenic Petunia plants

Agrobacterium strain LBA4404 transformants were used to transform *Petunia hybrida* using leaf discs as described by Horsch et al. (1985). After shoot and root induction on kanamycin selection media, plants were transferred to soil in the greenhouse.

### Example 8:

### Histochemical GUS assay

Different plant parts of Kanamycin-resistant plants transformed with the pBNEP1 construct were analysed for the distribution of β-glucuronidase activity (GUS) using the method described by (Jefferson et al., 1987). In transgenic plants with high expression levels diffusion of reaction products to other tissues was observed. To avoid this spreading a modified GUS assay was used. Briefly, tissues were pre-treated with 90% cold acetone at -20°C for 1 h, then rinsed three times 20' with 100 mM phosphate buffer containing 1 mM potassium ferricyanide. After this treatment the standard GUS assay was performed with the modification that ferricyanide was excluded from the reaction mixture.

### Example 9:

### Results histochemical GUS assay

In very young flowers (<1,4 cm) no blue staining was observed, in flowers of 2-4 cm weak blue staining of the nectaries was observed. In flowers of (4-6 cm) strong blue staining was observed in the nectaries (figure 6B) and in a very restricted region of the upper part of the anther filaments (Figure 6C). GUS expression was highest in the outer cell layers of the nectary parenchyma. In cross sections of the anther filaments GUS expression was observed in all cells except in the xylem of the inner vascular bundle.

### Example 10:

### Protein analysis of heather honey and nectar

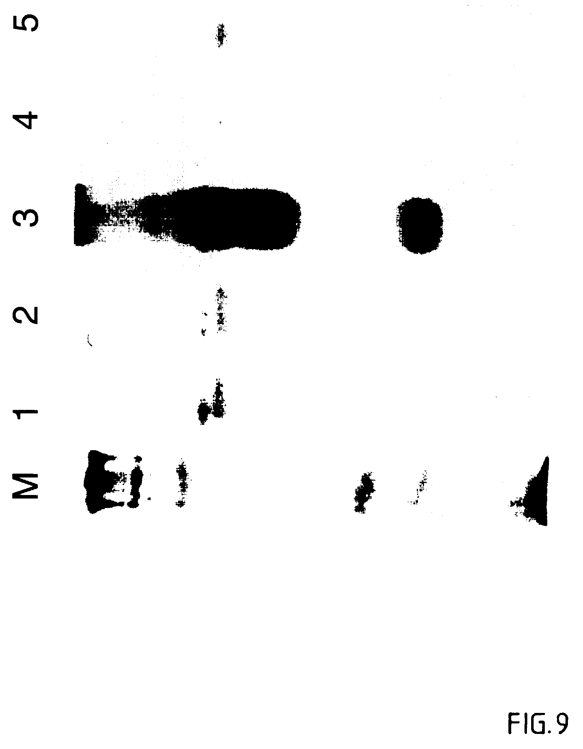

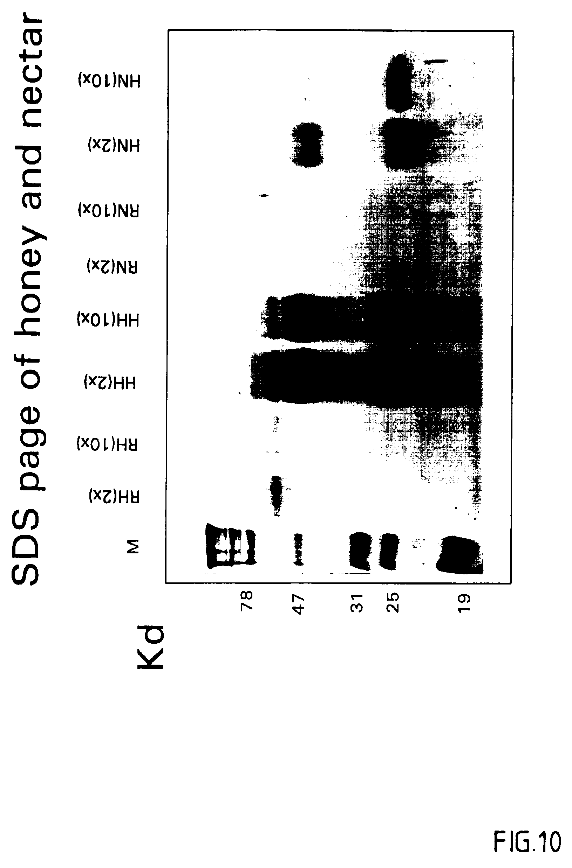

Samples of pure heather honey, together with samples of rapeseed, clover, wattle bark and lavender honey were diluted, dialysed and loaded on a 12% SDS page gel (Laemmli, 1970). All honey samples showed several identical high molecular weight protein bands. Heather honey contained 2 unique protein bands of 29 and 50 kDa (Figure 9). The proteins were named CVH29 and CVH50 (CVH stands for *Calluna vulgaris* honey). To determine the origin of the proteins, nectar and honey samples of rapeseed and heather were prepared and loaded on a 12% SDS page gel. The high molecular weight protein bands of around 70 kDa that are present in all honey samples were not observed in rapeseed or heather nectar (Figure 10). These proteins are added by honeybees during honey processing. Proteins CVH29 and CVH50 are present in heather honey and heather nectar, but not in nectar of rapeseed. Therefore, it was concluded that CVH29 and CVH50 are secreted in nectar of heather and can be recovered from honey derived from this nectar. The protein concentration in the heather honey we tested was around 0.5%.

### Example 11:

### N-terminal sequence analysis of CVH29 and CVH50

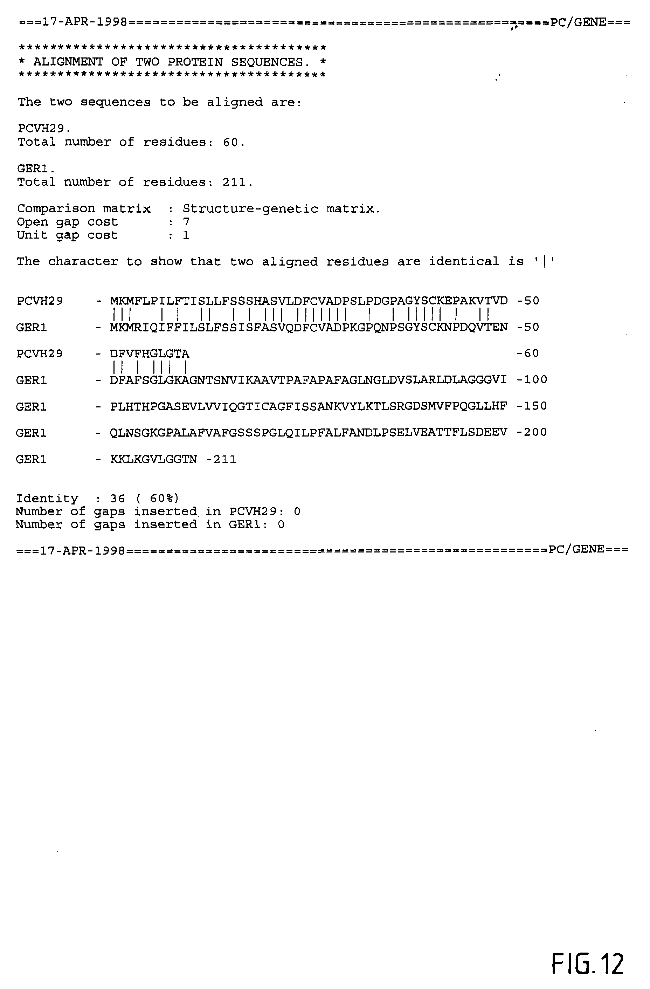

Honey samples were loaded on an SDS PAGE gel and after electrophoreses the gel was blotted on a PVDF membrane. After staining the CVH29 and CVH50 bands were cut out from the blot and N-terminal sequencing was performed on both proteins. The N-terminal sequence of CVH50 is: SVLDFCVADPSLPDGPAGYSCTEPSTVTSQDF. The N-terminal sequence of CVH29 is: SVLDFCVADPSLPDGPAGYSCKEPAKVTVDDFVFHGLGTA. A gene bank homology search (BLAST) showed high amino acid sequence homology (63%) with germin-like proteins isolated from Arabidopsis (Figure 12).

### Example 12:

### Identification signal sequence of CVH29

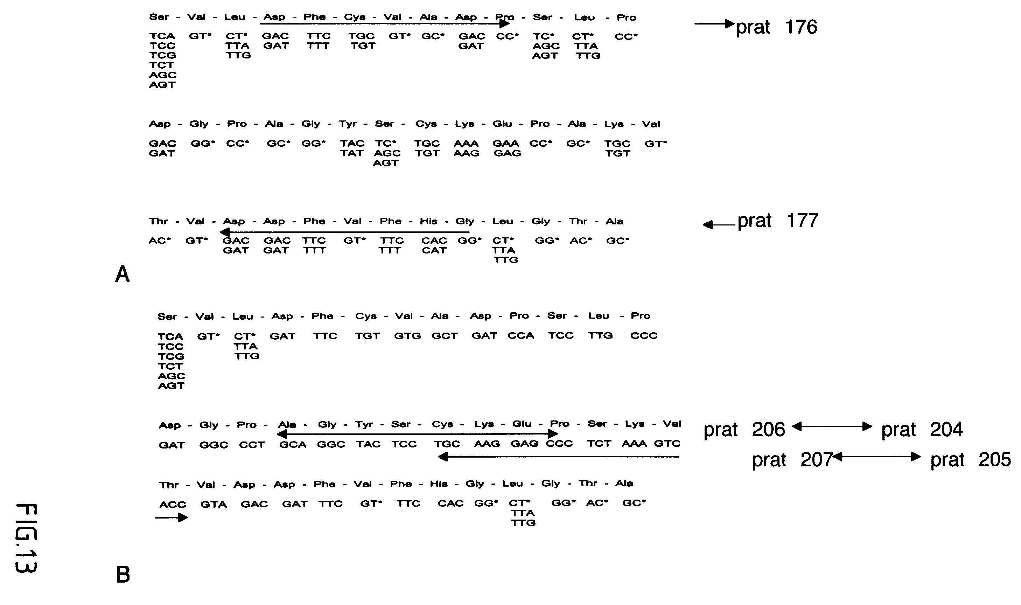

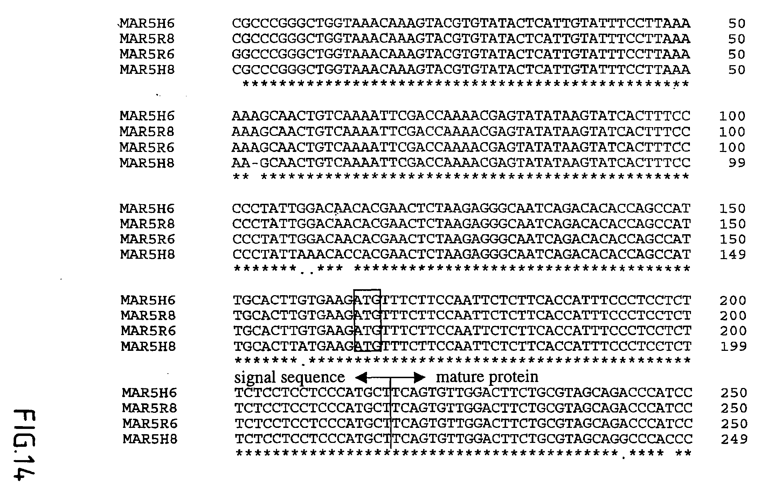

Because the germin-like protein CVH29 is excreted in heather nectar it was expected that part of the cDNA encodes a signal sequence. Based on the N-terminal amino acid sequence, degenerated primers were designed. The sequence of the forward primer prat 176 is: 5'-gayttytgygtngcngaycc-3' (y= c or t, n= c, t, a or g). The sequence of the reversed primer prat 177 is: ccrtgraanacraartcrtc (r= g or a). A PCR reaction performed on genomic DNA of heather yielded a 99 bp DNA fragment. The fragment was sequenced and two reversed, gene-specific 5' primers were designed to clone the 5' cDNA by "Marathon cDNA racing" using the kit and protocol of Clontech laboratories (protocol PT1115-1, Clontech Palo Alto USA). The sequence of gene-specific primer prat 207 that was used is: 5'-ggtgactttagagggctccttgc-3', the sequence of gene-specific nested primer prat 206 is:
5'-gctccttgcaggagtagcctgc-3' (Figure 13). RNA was isolated from open flowers of heather and mRNA was prepared using the Pharmacia quickprep micro mRNA kit. After cDNA synthesis and adapter ligation a PCR reaction was performed, using the adapter primer AP1 and the gene-specific primer prat 207. The PCR product was used for a second PCR, using adapter primer AP2 and the nested gene-specific primer prat 206. A single fragment of around 300 nucleotides was obtained and cloned in a PMOSBlue T-vector. Four clones were sequenced. Figure 14 shows that three clones were identical and one clone had two different nucleotides in the untranslated 5' region. A putative signal sequence of 17 amino acids was identified between the ATG start codon and the first codon of the mature protein CVH29 that was identical in all four clones. The nucleotide sequence of the putative signal sequence (SEQ ID NO:6) is:
5'-atgtttcttccaattctcttcaccatttccctcctcttctcctcctcccatgct-3'.

### Example 13:

### Construction of an expression cassette for excretion of proteins in nectar

To clone the *NEC1* promoter into a PMOSBlue vector a PCR reaction was carried out on pMA5-10 (example 5) using the forward primer prat 247 and the reversed primer prat 248 (Fig. 7). Prat 247 contains an extra Pst1 restriction site. The *Nde*I restriction site of prat 248 coincides with the ATG translation start of *NEC1*. The nucleotide sequence of prat 247 is: 5'-ggctgcaggagtgttctttgatagaatg-3', the nucleotide sequence of prat 248 is: 5'-cgccatatgtttttttatggaagcccc-3'. Gene-specific regions are underlined. A 1,8 kb promoter fragment was obtained and cloned into a pMOSBlue vector, yielding the plasmid pNECP.

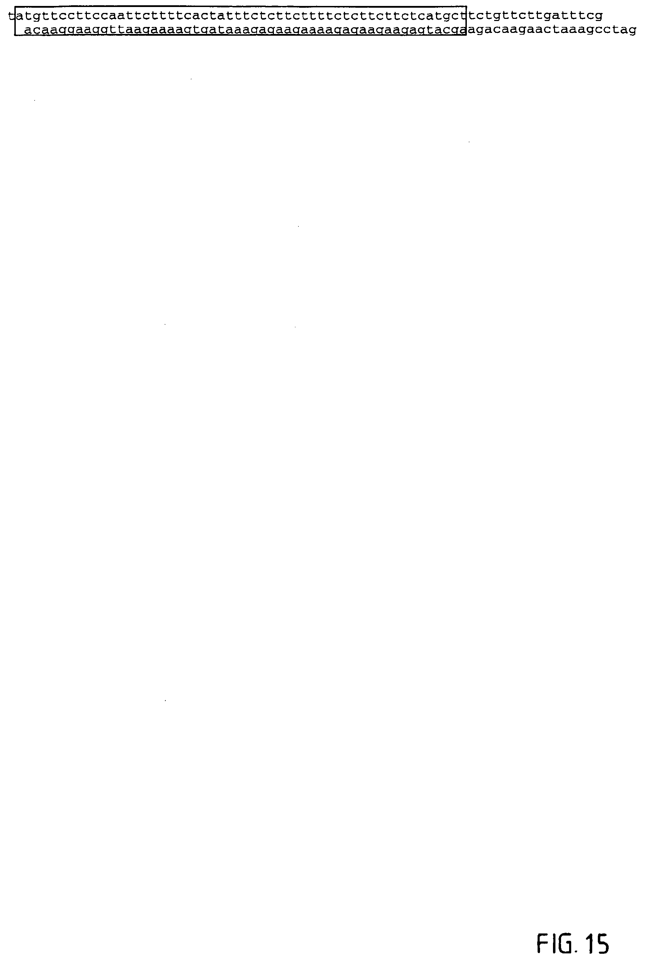

A DNA molecule encoding the signal sequence CVSP as depicted in SEQ ID NO:6 was produced by synthesis and subsequent annealing of two oligo molecules prat 245 and prat 246. The sequence of prat 245 is: 5'tatgttccttccaattcttttcactatttctcttcttttctcttcttctcatgcttctgttcttgatttc'3, the sequence of prat 246 is: 5'gatccgaaatcaagaacagaagcatgagaagaagagaaaagaagagaaatagtgaaaagaattggaaggaaca'3. The region encoding the signal sequence CVSP is underlined. To ensure correct cleavage of the signal peptide, the linkers were extended with the coding region for the first five amino acids of the mature germin-like protein (Fig. 13). The codon usage of the signal peptide sequence was optimised for Arabidopsis. By addition of a BamHI restriction site at the 3' end, 2 extra amino acids were linked in frame to the mature protein. The resulting DNA molecule is shown in Figure 15. The fragment was ligated into a Nde1/BamH1 cut PMOSBlue vector, yielding the plasmid pCVSP.

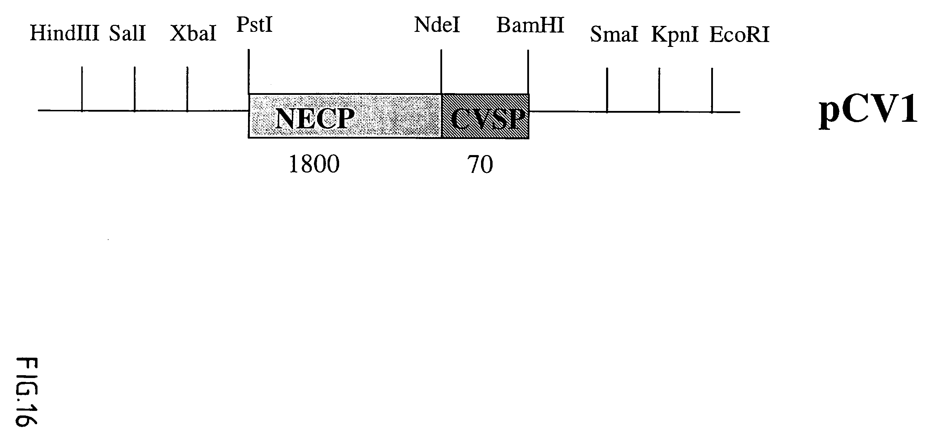

pNECP was digested with NdeI and PstI to release the *NEC1* promoter fragment which was cloned into the Pst1/Nde1 cut pCVSP, yielding the plasmid pCV1. A schematic representation of pCV1 is given in Figure 16.

A 250 bp long fragment containing the NOS terminator sequence (NOST) was obtained by PCR, using the forward primer prat 251 and the reversed primer prat 252 on DNA of pRAP 33, which is a pUC 19 derived plasmid. Prat 251 adds a SacI and XhoI site, prat 252 adds a SmaI and EcoRI site. The sequence of prat 251 is: 5'-gggagctcgagtcgttcaaacatttggcaataaag-3'. The sequence of prat 252 is: 5'-cgaattcccgggatctagtaacatagatgacac-3'. The NOST-specific regions are underlined. The PCR product was cloned into pCR-Script™ Amp SK(+) Cloning Kit (Catalog 21188-21190, Stratagene La Jolla USA), yielding the plasmid pCR-NOST. pCR-NOST was digested with Sac1 and EcoR1 and the resulting fragment was cloned into the pUC 19 (ClonTech), derived plasmid pUCAP yielding the plasmid pCVNOS.

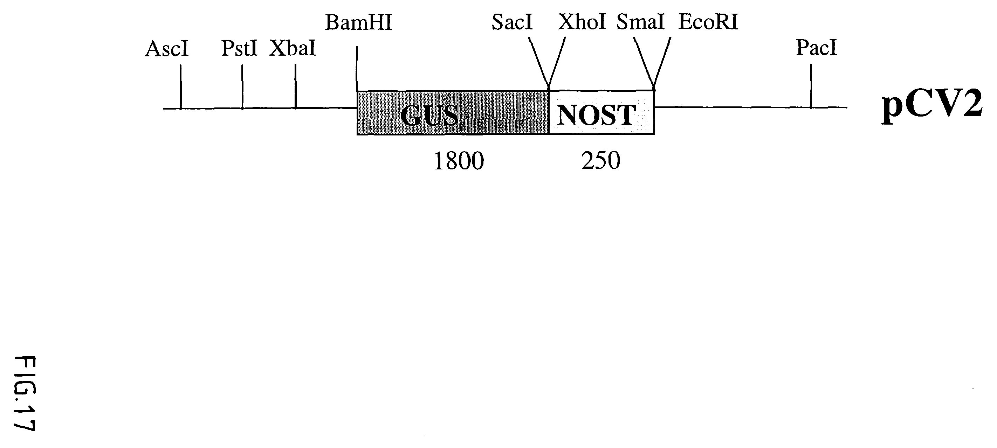

The plasmid pGUSN358 was purchased from Clontech (catalog 6030-1) containing the reporter gene GUS in pUC 119, modified to destroy the N-linked glycosylation site within the 1.814 Kb GUS coding sequence. A PCR reaction was carried out with gene-specific primers prat 249 and prat 250, yielding a fragment that contains the GUS gene coding region and a BamH1 restriction site at the 5'end and a Sac1 restriction site at the 3'end. The sequence of prat 249 is: 5'-ccggatccatgttacgtcctgtagaaacc-3'. The sequence of prat 250 is: 5'-gggagctcccaccgaggctgtag-3'. The GUS specific regions are underlined. Subsequently, the PCR fragment was digested with BamHI and SacI and ligated into the BamHI/SacI cut plasmid pCVNOS, yielding the plasmid pCV2. A schematic representation of pCV2 is given in Figure 17.

pCV1 is digested with PstI and BamHI and the resulting fragment is cloned into the PstI/BamHI cut plasmid pCV2, yielding the plasmid pCV3. A schematic representation of pCV3 is given in Figure 18. pCV3 is digested with AscI and SmaI and the resulting fragment is cloned into a derivative of the binary plasmid pBIN, yielding the binary plasmid pBCV3. pBCV3 was transferred from *Escherichia coli* to the *Agrobacterium tumefaciens* strain LBA4404 and C58pMP90 by electroporation. The transformed Agrobacterium strain was used to transform Arabidopsis and petunia.

### Example 14:

### Protein production in nectar

Using the *GUS* reporter gene, GUS activity in nectar of transgenic plants was measured according to the method as described by Jefferson et al., (1987). Briefly, the assay was carried out by measuring the amount of methyl umbelliferone (MU) produced by GUS fluorometrically by emission of light of 455 nm. The absolute emission was corrected for artificial quenching using an internal standard of 1nM MU (Angenent et al., 1993).

### Example 15:

### Feeding experiments with honeybees

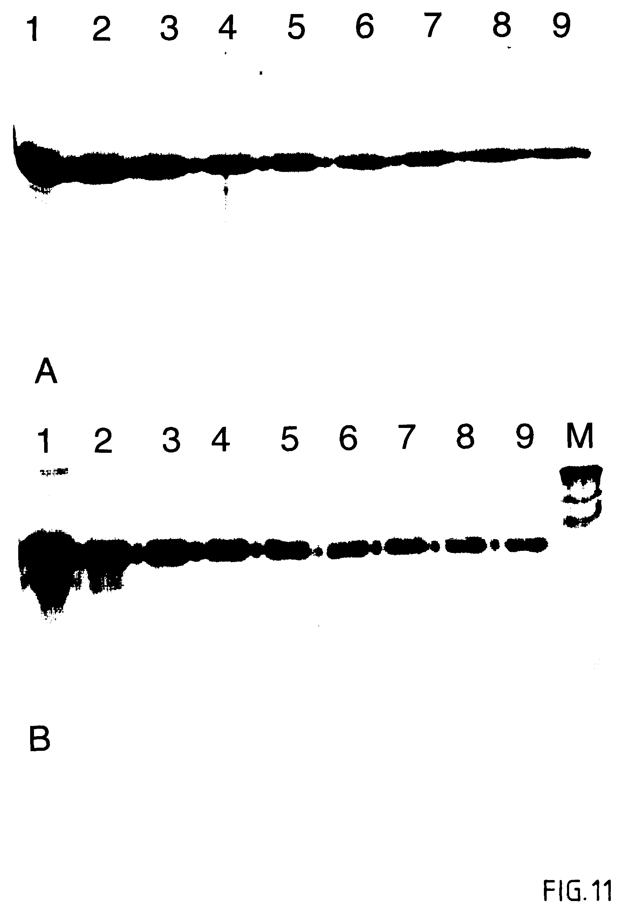

In September 1996 a beehive located outside was supplied with a 25% sucrose solution supplemented with 2% BSA (bovine serum albumin). After 3 weeks the bees had consumed 15 litters of the feeding solution and honey was harvested from the hive. Although the flowering season had mostly past, bees still foraged on flowers to collect nectar outside. Therefore, the honey produced during this period is derived from a mixture of the feeding solution and nectar from flowers. An SDS page protein gel was loaded with dialysed honey samples and sugar/BSA solutions. Figure 11 shows that the protein band of BSA was present in all the samples tested and no qualitative changes were observed in the honey samples compared to the sugar/BSA solutions. The BSA concentration in honey was 1.5 times higher than in the feeding samples, demonstrating that protein is concentrated in honey. Honeybees that foraged on the sugar/BSA solution did not show any aberrant behaviour and the colony developed normally.

### Example 16:

### Process of honey production from transgenic plants

Twohundred and fifty transgenic plants that each produce recombinant protein in nectar were grown in a greenhouse of 25 square meters. The facilities were adjusted according to the safety rules according to European law, including safety measures to prevent in- or outflow of insects. A beehive adjusted for small populations, containing around 200 worker honeybees and a queen, was placed in the greenhouse when the plants were flowering. When a queen is present, she will start laying eggs and larvae will come out. The presence of brood stimulates the bees to collect nectar and process it into honey. After 2-3 weeks bees processed the nectar into honey and stored in sealed cells of the honeycomb. Under the described conditions the amount of honey that can be harvested is 250-1000 grams.

### Example 17:

### Ablation of nectaries

By introducing the highly sensitive Rnase BARNASE in plant cells, under the control of a tissue-specific promoter, cell ablation can be achieved in very specific tissues or organs. Ablation of nectaries can be applied to decrease the attractiveness of plants for pest insects that forage on the nectar that is secreted by nectaries. In addition, plants without nectaries can be obtained that are more resistant to bacterial and fungal infections. An example is given for the ablation of nectary tissue by expressing bacterial BARNASE in nectaries, using the NEC1 promoter.

Plasmid DNA of pNEP1 (example 6) was digested with KpnI and NcoI to release the 1800 bp *NEC1* promoter fragment. The purified promoter fragment was ligated into a pWP90 derived vector, upstream of the BARNASE-BARSTAR bacterial operon construct (Hartley, 1988). The construct contains a 35SCaMV terminator of polyA signal cauliflower mosaic virus terminator sequence downstream of the BARNASE-BARSTAR operon. The resulting plasmid pWP126 was digested with KpnI/ XhoI to release the *NEC1*-promoter/BARNASE-BARSTAR/CamVpolyA fragment and this fragment was ligated into a pBIN-derived vector pBIN Plus. The recombinant vector was transferred via *Agrobacterium tumefaciens* (LBA4404) to petunia variety W115. Transgenic petunia plants were selected with flowers without nectaries or underdeveloped nectaries.

Many promoters are less specific than can be concluded based on promoter/GUS expression is concluded. Because the bacterial BARNASE is highly cytotoxic at very low concentrations it can be preferred to protect other plant tissues by expression of a ribonuclease inhibitor gene under the control of a weak, constitutive promoter (e.g. NOS promoter) or a tissue-specific promoter that is not active in the tissues where cell ablation is to be achieved (Mariani et al., 1992, Beals et al., 1997).

### Example 18:

### Ectopic nectary development

MADS box genes regulate floral meristem and floral organ identity. Ectopic expression of MADS box genes can change the developmental fate of floral organs or cells. Transgenic petunia plants ectopically expressing FBP11, an ovule-specific MADS box gene, develop ovule-like structures on sepals and petals ( Colombo et al., 1995). *FBP15* is a nectary-specific MADS box gene, involved in the molecular regulation of nectary development. In petunia nectaries develop at the base of the carpel. Ectopic expression of *FBP15* in petunia may result in the development of nectaries on other organs of the flower or on vegetative parts of the plant. An example is given of a gene construct that, when transformed to a plant, results in ectopic expression of *FBP15*.

*FBP15* was amplified using a 5' primer that hybridises with *FBP15* sequences just upstream of the ATG translation start site and a 3' primer that hybridises with *FBP15* sequences just downstream the translation stop site. The 5'primer contains a NcoI recognition site, the 3'primer contains a BamHI recognition site. After the sequence was confirmed, the amplified *FBP15* fragment was inserted as a BamHI/NcoI fragment into the binary vector pCPO31. This binary vector was derived from pPCV708, as described by Florack et al. (1994), and contains three expression cassettes with a multiple cloning site between the left and right T-DNA borders. The cDNA was cloned in sense orientation between a modified CaMV 35S promoter and the nopaline synthase terminator sequence. The chimerical gene construct was transferred via Agrobacterium GV3101 to petunia variety W115, using the transformation method as described in example 7. Transgenic petunia plants were selected that show ectopic nectary development.

### Example 19:

### Modification of sugar composition and nectar secretion

Although sugar content of nectar from different petunia W115 flowers shows some variation, the ratio between hexoses and sucrose is very stable. Down-regulation or up-regulation of genes involved in the establishment of the ratio between hexoses and sucrose in nectar will therefore modify nectar composition. An example is given for anti-sense expression of a petunia-derived invertase gene.

PCR primers were designed that hybridise with the cDNA of an invertase gene cloned from *Solanum tuberosum*. The 5' primer 5'-AAGGACTTTAGAGAGACCCGACCACTGCTGG-3'and the 3' primer 5'-AAATGTCTTTGATGCATAATATTTCCCATAATC-3' were used for a PCR reaction on genomic DNA of petunia to yield a fragment of around 420 bp. The fragment was sequenced and cloned into a pMOSBlue vector to used as a probe to screen a petunia nectary-specific cDNA library. Hybridizing phage plaques were purified and cDNAs were retrieved by in vivo excision as described in example 2. The expression of the cDNA's was determined by Northern blotting as described in example 3 and the sequence of a nectary-specific invertase was determined as described in example 2. The invertase gene was amplified using a 5' primer that hybridises with sequences just upstream of the ATG translation start site and a 3' primer that hybridises with sequences just downstream of the translation stop site. Extra restriction enzyme recognition sites were generated to allow cloning of the cDNA in sense (overexpression) or antisense direction into the binary vector pCPO31 as described in example 18. The chimerical gene constructs are transferred via Agrobacterium GV3101 to petunia variety W115, using the transformation method as described in example 7. Transgenic petunia plants were selected that exhibit modified sugar composition in nectar.

### Example 20

### Modification of plant development

A DNA which is the *NEC1* gene or a homologous gene is introduced into a plant cell, the said DNA being induced by promoter elements controlling the expression of the introduced DNA in such a way that transcription produces sense RNA. Plants were regenerated from the transgenic cells as described in example 7. Plants that ectopically express the *NEC1* gene exhibited modified leaf morphology and modified sugar composition. Furthermore, plants that ectopically express the *NEC1* gene showed a delay in flowering time.

### REFERENCES:

Angenent G.C., Franken J., Busscher M., Colombo L. and van Tunen A.J., 1993. Petal and stamen formation in petunia is regulated by the homeotic gene *fbp*1. Plant J. 4, 101-112.
Angenent G.C., Franken J., Busscher M., Weiss D. and van Tunen A.J., 1994. Co-suppression of the petunia homeotic gene *fbp*2 affects the identity of the generative meristem. Plant J. 5, 33-44.
Baker H.G. and Baker I., 1982. Floral nectar constituents in relation to pollinator type. In: Handbook of experimental pollination biology (ED. by CE Jones and RJ Little), pp 117-141. Scientific and Academic Edition, Van Nostrand Reinhold, New York.
Beals T.P. and Goldberg R.B. (1997). A novel cell ablation strategy blocks tobacco anther dehiscence. The Plant Cell 9, 1527-1545.
Bevan M. (1984). Binary *Agrobacterium* vectors for plant transformation. Nucl. Acids Res. 12, 8711-8721
Butler C.G. (1962). The world of the honeybee. London: Collins.
Canãs L.A., Busscher M., Angenent G.C., Beltran J-P and van Tunen A.J. (1994). Nuclear localisation of the petunia MADS box gene protein *FBP1*. Plant J. 6, 597-604.
Colombo L., Franken J., Koetje E., van Went J., Dons H.J.M, Angenent G.C. and van Tunen A.J. (1995). The petunia MADS Box gene *FBP11* determines ovule identity. The Plant Cell 7, 1859-1868.
Crailsheim k., Hrassnigg N., Lorenz W and Lass A. Protein consumption and distribution in a honeybee colony (Apis mellifera carnica Pollm). Apidologie 24 (5), 510-511.
Custers J.B.M., Oldenhof, Schrauwen J.A.M., Cordewener J.H.G., Wullems G.J. and Lookeren Campagne M.M. (1997). Analysis of microspore-specific promoters in transgenic tobacco. Plant molecular biology 35, 689-699.
Davis A.R., Peterson R.L. and Shuel R.W. (1986). Anatomy and vasculature of the floral nectaries of *Brassica napus* (Brassicaceae). Can. J. Bot. vol. 64, 2508-2516.
Dumas B., Freyssinet G. and Pallett E. (1995). Tissue-specific expression of germin-like oxalate oxidase during development and fungal infection of barley seedlings. Plant Physiol. 107: 1091-1096.
Fahn A. (1979). Ultrastructure of nectaries in relation to nectar secretion. Amer. J. Bot. 66(8), 977-985.
Florack D.E.A, Dirkse W.G., Visser B., F. Heidekamp and Stiekema W.J. (1994). Expression of biologically active hordothionins in tobacco: Effects of pre- and pro-sequences at the amino and carboxyl termini of the hordothionin precursor on mature protein expression and sorting. Plant Mol. Biol. 24. 83-96.
Hartley R.W. (1988). Barnase and Barstar: Expression of its cloned inhibitor permits expression of a cloed ribonuclease. J. Mol. Biol. 202, 913-915
Horsch R.B., Fry J.E., Hofman N.L., Eichholz D., Rogers S.G. and Fraley R.T. (1985). A simple and general method for transferring genes into plants. Science 227, 1229-1231.
Huang H., Tudor M., Su T., Hu Y. and Ma H. (1996). DNA binding properties of two Arabidopsis MADS domain proteins: Binding consensus and dimer formation. The Plant Cell 8, 81-94.
Jefferson R.A., Kavanagh T.A. and Bevan M. (1987). GUS fusion: J-glucuronidase as a sensitive and versatile gene fusion marker in higher plants. EMBO J. 6, 3901-3907.
Laemmli U.K. (1970). Cleavage of structural proteins during the assembly of the head bacteriophage T4. nature 227, 680-685.
Lane B.G., Dunwell J.M., Ray J.A., Schmitt M.R. and Cuming A.C. (1993). Germin, a protein marker of early plant development, is an oxalate oxidase. The Journal of Biological Chemistry 268 (17), 12239-12242.
Mariani C., Gossele V., De Beuckeleer M., De Block M., Goldberg R.B., De Greef W. and Leemans J. (1992). a chimaeric ribonuclease-inhibitor gene restores fertility to male sterile plants. Nature 357, 384-387.
Martini M., Schmid A. and Hess D. (1990). Antibiotics, and amino acids in nectar of *Rhododendron* and *Piptanthus* species from Nepal. Bot. Acta 103, 343-348.
Mizukami Y., Huang H., Tudor M. and Ma H. (1996). Functional domains of the floral regulator AGAMOUS: Characterisation of the DNA binding domain and analysis of dominant negative mutations. The Plant Cell 8, 831-845.
Tsuchimoto S., van der Krol A.R. and Chua N.-H, 1993. Ectopic expression of pMADS3 in transgenic petunia phenocopies the petunia *blind* mutant. Plant Cell 5, 843-853.
Verwoerd T.C., Dekker, B.M.M. and Hoekema A. (1989). A small-scale procedure for the rapid isolation of plant RNAs. Nucl. Acids Res. 17, 2362.
Zer H. and Fahn A. (1992). Floral nectaries of *Rosmarinus officinalis* L. Structure, Ultrastructure and Nectar Secretion. Annals of Botany 70, 391-397.

Sequences:
SEQ ID NO:1 amino acid sequence NEC1

1 MAQLRADDLS FIFGLLGNIV SFMVFLAPVP TFYKIYKRKS SEGYQAIPYM

51 VALFSAGLLL YYAYLRKNAY LIVSINGFGC AIELTYISLF LFYAPRKSKI

101 FTGWLMLLEL GALGMVMPIT YLLAEGSHRV MIVGWICAAI NVAVFAAPLS

151 IMRQVIKTKS VEFMPFTLSL FLTLCATMWF FYGFFKKDFY IAFPNILGFL

201 FGIVQMLLYF VYKDSKRIDD EKSDPVREAT KSKEGVEIII NIEDDNSDNA

251 LQSMEKDFSR LRTSK

SEQ ID NO:2 amino acid sequence of FBP15

MGRGKIEIKRIENNRTNRQVTFCKRRNGLLKKAYELSVLCDAEVALIVFSSRGRLY-
EYANNSVKATIDRYKKASSDSSNTGSTSEANTQFYQQEAAKLRVQIGNLQNSNRNML-
GESLSSLTAKDLKGLETKLEKGISRIRSKKNELLFAEIEYMRKREIDLHNNNQMLRA-
KIAESERNVNMMGGEFELMQSHPYDPRDFFQVNGLQHNHQYPRQDNMALQLVU

SEQ ID NO:3 amino acid sequence CVSP

MFLPILFTISLLFSSSHA

SEQ ID NO:4 Nucleotide sequence NEC1

1 TCGAGCGGCC GCCCGGGCAG GTATTCAACA AGAGTATTCA CCACTTGAAC
51 TCAAAAGGGG CTTCACTAAA AAAAAATCAT GGCGCAATTA CGTGCTGATG
101 ACTTGTCTTT CATATTTGGC CTTCTTGGTA ATATTGTATC ATTCATGGTC
151 TTCCTAGCAC CCGTGCCAAC ATTTTACAAA ATATATAAAA GGAAATCATC
201 AGAAGGATAT CAAGCAATAC CATATATGGT AGCACTGTTC AGCGCCGGAC
251 TATTGCTATA TTATGCTTAT CTCAGGAAGA ATGCCTATCT TATCGTCAGC
301 ATTAATGGCT TTGGATGTGC CATTGAATTA ACATATATCT CTCTGTTTCT
351 CTTTTACGCG CCCAGAAAGT CTAAGATTTT CACAGGGTGG CTGATGCTCT
401 TAGAATTGGG AGCCCTAGGA ATGGTGATGC CAATTACTTA TTTATTAGCA
451 GAAGGCTCAC ATAGAGTGAT GATAGTGGGA TGGATTTGTG CAGCTATCAA
501 TGTTGCTGTC TTTGCTGCTC CTTTAAGCAT CATGAGGCAA GTAATAAAAA
551 CAAAGAGTGT AGAGTTCATG CCCTTCACTT TATCTTTGTT CCTCACTCTC
601 TGTGCCACTA TGTGGTTTTT CTATGGGTTT TTCAAGAAGG ACTTTTACAT
651 TGCGTTTCCA AATATACTGG GCTTTCTATT CGGAATCGTT CAAATGCTAT
701 TATATTTTGT TTACAAGGAT TCAAAGAGAA TAGATGATGA AAAATCTGAT
751 CCTGTTCGAG AAGCTACAAA ATCAAAAGAA GGTGTAGAAA TCATTATCAA
801 CATTGAAGAT GATAATTCTG ATAACGCATT GCAGTCCATG GAGAAGGATT
851 TTTCCAGACT GCGGACATCA AAATAAGCAA GAAGATGATC AAAAAATGAC
901 AAAGCTAAGG AGTTTGAAGT AAGGCAAGGA ACTTGACACT GAATATCTAA
951 GCTAATTAGC AAGACTTTAG CAGCTTGTAA TATTTAGTGT TTGTGAGGTG
1001 TTACCTTATA ATTAGCTTGT AGCATAGCCT TCCCACTAAT AATTCTGCTT
1051 AGCGAATCTT ATATATGGGA AATACTTACA CTAGTATGCA TCTTCTATAT
1101 ACATGTTTGG CACTTGACTA TACATAGAAA AATTAACAAG CATTTCTCAC
1151 CTCAATTTGT CACTTACTTA TAAGTAGCTG AATAATATAA TGCAATTTTC
1201 ACCCC

SEQ ID NO:5 Nucleotide sequence FBP15

1 TCTGAATACAAGCTGTGTGTGTAGAGAGATTTCATAAAGACAGCAAACAT

51 CCCTTCTTTTGTTCTGTTTTAAAAGTTCCCTTCTTCAACCAGCTCTTTT

101 CCTCATCAGGGTAAGTTGCAAATAAAGGGGATGTTCCAGAATCAAGAAGA

151 GAAGATGTCAGACTCGCCTCAGAGGAAGATGGGAAGAGGAAAGATTGAGA

201 TTAAGAGGATTGAAAATACAACAAATCGTCAAGTCACTTTCTGTAAGAGA

251 AGAAATGGGTTGCTTAAAAAAGCTTATGAACTTTCTGTTCTTTGTGATGC

301 TGAAGTTGCTCTCATCGTTTTCTCAAGCCGTGGCCGCCTCTATGAATATG

351 CTAACAACAGTGTGAAGGCAACAATTGATAGATATAAGAAAGCATCCTCA

401 GATTCCTCCAACACTGGATCTACTTCTGAAGCTAACACTCAGTTTTATCA

451 ACAAGAAGCTGCCAAACTCCGAGTTCAGATTGGTAACTTACAGAACTCAA

501 ACAGGAACATGCTAGGCGAGTCTCTAAGTTCTCTGACTGCAAAAGATCTG

551 AAAGGCCTGGAGACCAAACTTGAGAAAGGAATTAGTAGAATTAGGTCCAA

601 AAAGAATGAACTCCTGTTTGCTGAGATTGAGTATATGCGAAAAAGGGAAA

651 TTGATTTGCACAACAACAATCAGATGCTTCGGGCAAAGATAGCTGAGAGT

701 GAAAGAAATGTGAACATGATGGGAGGAGAATTTGAGCTGATGCAATCTCA

751 TCCGTACGATCCAAGAGACTTCTTCCAAGTGAACGGCTTACAGCATAATC

801 ATCAATATCCACGCCAAGACAACATGGCTCTTCAATTAGTATAAGTTTAT

851 AATAAAATGCATGGTTTGAAGCACTCTGATTGTGGTGGATTTGGATTATG

901 TATAAGGGAGTGCAGGCCATTTGCCAATTATTGAAAGGTACTCAAACAGG

951 AAGTTGAAGAAGTTCATCATCTCTCTCATCTATATGTCTTAACAAAAGTC

1001 TTAGCTTATGGACTCTAAAACAAAGACTTAATTTAACATATAAATATAAT

1051 TGTGTAATGCTGTTGTATTGTATGGTATGTATCCAAAAACATTAATAACC

1101 TATCTTTTTCTTCAAATTATGTCTCCTTTGATACAAACTACTAACATATT

1151 TTCTTAT

SEQ ID NO:6 Nucleotide sequence CVSP

ATGTTTCTTCCAATTCTCTTCACCATTTCCCTCCTCTTCTCCTCCTCCCATGCT

SEQ ID NO:7 Nucleotide sequence NEC1 promoter

1 CCTAGGAGAAATCAAGCCTACTCTTAAGATGGATGACTCACTTGCCCCGA

51 TGGTAAGGTGAAGGATCTGTTGATTAGAGTTGGGAAGTTCATGTTCTCTG

101 CTGATTTTATTATTCTAGACTATGAAGAGGACCAAGAAGCTCCAATAATT

151 TTGGGAAGAGCATTCTTAATCACATCGATGGCAATTATTGACATGGAACT

201 TGGGGAGATGACTGTGAGAGCGCATGGAGAAAAGGTTACTTTCAAGGTTT

251 ATAATAAAAAGGATCATATGGCTAAGTTTGAAGAGTGTTCTTTGATAGAA

301 TGTGTCAGACGAGAACATGAAAGTAAACCGAAAGAGGTGTTTGAGCGGAA

351 TGTAGAACAAAGTGACCACGGCACAATAATTGACAAGTTGAAGGAAAATT

401 CACCTAAAGGAAGGAAGAAGACAAAAGTTCGTCGTAACAAGAGGAGACGT

451 AAATGCTGGAAGTGAGCTTAAAGGTGTTGTCGTACTACGACGTTAACTAA

501 GGCGCTTGTCGGGAGGCAACCCTAGCTTTGTATGTAAATGTAAAAGTAAA

551 AAATATATATATAGAAAAAGGAAAATACAAAAAGAGTCGTGCCGCGACGT

601 TAAATCAAGCGCTTGTTGGAAGGCAACCCAATTTTTATTGTTTTAGTTGT

651 TTTACTTATTTAGTATTACGTAGTTTCTTGTTGTTTTTGTAGGGCTCGGG

701 ACTTTCGGAAGGTGAGGTAATTTCAAGGCATCGCGGTGTGTATTGCAGCG

751 AGGTAAGTGTAAGAGTTGAGTTGGAAGCGTTTGGCCAAGTGTTGCACCGT

801 GAGAGGCTTTCAACCTGTTGCGACACGTGAAAAATTAAGAGCCAGATCTG

851 CTACATTAGCACTGAAGCATCGCTTGGCCAATAGCTTGGAATGGAAGCAA

901 GAATTCAAACCAAAATCAGAAACGCCACAAGAGATGTGTCGCACACTGCA

951 AAGCTTTGTGCAAACTAGTGAACGCAGAAATAGAAATGCTACAGCCCATG

1001 CGTCGCTTGGCTTATGGCAGGCAGCAAAAATTCAGCAGCAAAACAGAAAC

1051 GCTGCGAGAAACGCGTCGCATACGCCATAGCTTTGTGTCAAACAGAACGT

1101 CCAGAAATTGAAAAGCTATAAGCCTGCGTCGCTTGGCTCATGGCGTGCAG

1151 ACTAGAAAAGCTCTAGCAGATGCGTCGCGTATTGTATAGCTTGGTGTGAA

1201 ACAGAAAGTTCGAAACTTGGAAAACGATAACCCAGCGTCGCCTCTTCAAC

1251 CGCGTCCAGGTAAGTTCAAGATTCTTACGGGTTGACCCATTAACCCATTG

1301 ATCGGCTGATTATAAACAATAAAACATCACCTTCAACTATCACATGATTT

1351 CATAAGTTTGACCTAGGATATTTATATATATATATATATATATACACAC

1401 ACACACCATTTCCAGCGATCTTACCTCATTTTTATTCAAACCATTTTTCT

1451 GCTTCAAAAGTTTAAATTATTAATATGATAAGTCATCCATAGTCAAACAA
1501 GATTTTCTATACTATTTTGTCCCTTGTAATTTTAAAAAAAAAATGAGCGA
1551 TGGTAAGATAAACATTGTTTGCAAGTGTACAATTTTAGTATATGCAAACC
1601 AACGCTTCTTCTTCCAACTATCACCTAAAACTACATCATTTATGGCGGGC
1651 GGACTAGACGTAGCCAAATATAAAAACGCAATGGCCATTCAGTTCATGTC
1701 ATTTTTATATCCTTCATCCAATAATATTACTCAAAATTGATGTACAGTTT
1751 GGTCTCTGATGTGCACTTTACTATACGTAATACGGAATTTACATTATAAT
1801 TAAAGAGAACTGTTCCACTAAATTTTAATGATTTAATTAATTTAACTCGG
1851 TTACTTGTATTATTATTATTGCTGTATTTGTTTGTCATTTGAATTTGGCA
1901 CCGCAGATTTTTGTATGCAATTAACCCTCATATATCTTTTGGCCAAATAA
1951 AGAAAAAGTCTGCATATTTCTTGCCAAACATTTATCATACTTTACCGAAT
2001 TCTTGTTTTTTGTTTCTCTGTTGTTGTTCTCCACTATAAATAACATTTGC
2051 AGTGAGTAAAGTTTCTTCAGGTCTCTTTTGTAGATTCAACAAGAGTATTC
2101 AGCACTTGAACTCAAAAGGGGCTTCACTAAAAAAAATCATG

SEQ ID NO:8 Nucleotide sequence FBP15 promoter

## Claims

**1.** An isolated DNA sequence from the promoter region upstream of a nectary-specific expressed sequence, which nectary-specific expressed sequence encodes a protein comprising the amino acid sequence given in SEQ ID NO: 1, or a homologous protein.

**2.** An isolated DNA sequence according to claim 1, wherein the nectary-specific expressed sequence has: a) a nucleotide sequence given in SEQ ID NO:4, or b) a nucleotide sequence obtainable by hybridisation with the nucleotide sequence of (a) or with a fragment of (a).

**3.** An isolated DNA sequence according to claim 1 or 2, obtained from a plant of *Petunia hybrida*, the sequence consisting essentially of the sequence given in SEQ ID NO:7, or a functional fragment thereof having promoter activity.

**4.** An isolated DNA sequence from the promoter region upstream of a nectary-specific expressed sequence, which nectary-specific expressed sequence encodes a protein comprising the amino acid sequence given in SEQ ID NO:2, or a homologous protein.

**5.** An isolated DNA sequence according to claim 4, wherein the nectary-specific expressed sequence has:
a) a nucleotide sequence given in SEQ ID NO:5, or
b) a nucleotide sequence obtainable by hybridisation with the nucleotide sequence of (a) or with a fragment of (a).

**6.** An isolated DNA sequence according to claim 4 or 5, obtained from a plant of *Petunia hybrida*, the sequence consisting essentially of the sequence given in SEQ ID NO:8 or a functional fragment thereof having promoter activity.

**7.** An isolated DNA sequence encoding a protein comprising the amino acid sequence given in SEQ ID NO:1, or a homologous protein, the expression of the DNA sequence being predominantly confined to the nectaries of a plant.

**8.** An isolated DNA sequence according to claim 7 having:
a) a nucleotide sequence given in SEQ ID NO:4, or
b) a nucleotide sequence obtainable by hybridisation with the nucleotide sequence of (a) or with a fragment of (a).

**9.** An isolated DNA sequence that results from the sequence shown in SEQ ID NO:4 by insertion, deletion or substitution of one or more nucleotides, including naturally occurring variations or variations introduced by targeted mutagenesis or recombination.

**10.** An isolated DNA sequence according to claim 7 having a nucleotide sequence given in SEQ ID NO:4, said sequence being produced by current DNA synthesis techniques.

**11.** An isolated DNA sequence encoding a protein comprising the amino acid sequence given in SEQ ID NO:2, or a homologous protein, the expression of the DNA sequence being predominantly confined to the nectaries of a plant.

**12.** An isolated DNA sequence according to claim 11, having:
a) a nucleotide sequence given in SEQ ID NO:5, or
b) a nucleotide sequence obtainable by hybridisation with the nucleotide sequence of (a) or with a fragment of (a).

**13.** An isolated DNA sequence that results from the sequence shown in SEQ ID NO:5 by insertion, deletion or substitution of one or more nucleotides, including naturally occurring variations or variations introduced by targeted mutagenesis or recombination.

**14.** An isolated DNA sequence according to claim 11 having a nucleotide sequence given in SEQ ID NO:5, said sequence being produced by current DNA synthesis techniques.

**15.** Any sequence that encodes a nectary-specific MADS box gene.

**16.** An isolated DNA sequence comprising the coding region for a signal peptide, wherein the information contained in the DNA sequence permits, upon translational fusion with a DNA sequence encoding a protein that is expressed in nectaries, targeting of the protein to nectar.

**17.** An isolated DNA sequence according to claim 16, comprising the nucleotide sequence given in SEQ ID NO: 6 obtained from a plant of *Calluna vulgaris*, or a nucleotide sequence obtainable by hybridisation with the nucleotide sequence given in SEQ ID NO:6.

**18.** A recombinant double-stranded DNA molecule comprising an expression cassette comprising the following constituents:
i) a promoter functional in plants,
ii) a DNA sequence coding for a protein as defined in any of claims 7 to 15 which is fused to the promoter sequence in sense or antisense orientation, and optionally
iii) a signal sequence functional in plants for the transcription termination and polyadenylation of an RNA molecule.

**19.** A recombinant double-stranded DNA molecule comprising an expression cassette comprising the following constituents:
i) a promoter functional in nectaries of plants,
ii) a DNA sequence coding for a protein which is fused to the promoter sequence in sense or antisense orientation, and optionally
iii) a signal sequence functional in plants for the transcription termination and polyadenylation of an RNA molecule.

**20.** A recombinant double-stranded DNA molecule comprising an expression cassette comprising the following constituents:
i) a promoter functional in nectaries of plants,
ii) a DNA sequence encoding a protein which is fused to the promoter,
iii) a DNA sequence encoding a signal peptide that targets the recombinant protein to nectar, which is translationally fused to the DNA sequence encoding the recombinant protein, and optionally
iv) a signal sequence functional in plants for the transcription termination and polyadenylation of an RNA molecule.

**21.** A recombinant double-stranded DNA molecule according to claim 19 or 20 wherein the promoter is as defined in any of claims 1-6.

**22.** A recombinant double-stranded DNA molecule according to claim 20 or 21 wherein the DNA sequence encoding a signal peptide is as defined in claim 16 or 17.

**23.** A process for producing a transgenic plant exhibiting excretion of a recombinant protein in its nectar, comprising:
i) introducing in a plant cell a recombinant double-stranded DNA-molecule as defined in any of claims 20 to 22, wherein the recombinant protein is excreted in nectar
ii) regenerating plants from the transgenic cell, and
iii) selecting transgenic plants.

**24.** A process for producing a transgenic plant exhibiting a modified nectar composition, comprising:
i) introducing in a plant cell a recombinant double-stranded DNA-molecule as defined in any of claims 19 to 22, wherein the recombinant protein interferes with metabolic pathways in the nectaries,
ii) regenerating plants from the transgenic cell, and
iii) selecting transgenic plants.

**25.** A process for producing a transgenic plant exhibiting a modified nectar secretion, comprising:
i) introducing in a plant cell a recombinant double-stranded DNA-molecule as defined in any of claims 19 to 22, wherein the recombinant protein interferes with sink strength of nectaries
ii) regenerating plants from the transgenic cell, and
iii) selecting transgenic plants.

**26.** A process for producing a transgenic plant exhibiting a modified nectary development, comprising:
i) introducing in a plant cell a recombinant double-stranded DNA-molecule as defined in claims 19 or 22, wherein the recombinant protein interferes with the development of nectaries
ii) regenerating plants from the transgenic cell, and
iii) selecting transgenic plants.

**27.** A process for producing honey from modified nectar of transgenic plants, comprising:
i) producing a transgenic plant by introducing in a plant cell a recombinant double-stranded DNA molecule as defined in any of claims 19 to 22, regenerating plants from the transgenic cell, and selecting modified plants exhibiting the excretion of nectar with a modified composition,
ii) allowing insects, preferably bees, to collect nectar from the transgenic plants and to process the nectar into honey.

**28.** A process for producing a recombinant gene product from honey, comprising:
i) producing a transgenic plant by introducing in a plant cell a recombinant double-stranded DNA molecule as defined in any of claims 20 to 22, regenerating plants from the transgenic cell, and selecting modified plants exhibiting excretion of the recombinant gene product in nectar,
ii) allowing insects, preferably bees, to collect nectar from the transgenic plants and to process the nectar into honey, and
iii) isolating and purifying the gene product from the honey.

**29.** A process for producing a metabolite from honey, comprising:
i) producing a plant that excretes this metabolite in nectar and which plant has been produced by current breeding and selection methods,
ii) allowing insects, preferably bees, to collect nectar from the selected plants and to process the nectar into honey, and
iii) isolating and purifying the metabolite from the honey.

**30.** Micro organisms containing DNA sequences according to one or more of claims 1 to 17.

**31.** Micro organisms containing recombinant DNA molecules according to any of claims 18 to 22.

**32.** A plant cell or plant cell culture transformed with one or more DNA sequences according to claims 1 to 17.

**33.** A plant cell or plant cell culture transformed with recombinant DNA molecules according to any of 18 to 22.

**34.** A plant consisting essentially of the plant cells of claims 32 or 33.

**35.** A transgenic plant obtained by the process of any of claims 23 to 26.

**35.** Seeds, tissue culture, plant parts or progeny plants derived from a transgenic plant according to claim 35.

**36.** Honey obtained from nectar from transgenic plants, which nectar has a modified composition.

**37.** Honey obtained from nectar from transgenic plants, which nectar comprises a recombinant gene product.


SEQUENCE LISTING

(1) GENERAL INFORMATION:

(i) APPLICANT:
(A) NAME: Center for Plant breeding and Reproduction
Research
(B) STREET: Droevendaalsesteeg 1
(C) CITY: Wageningen
(D) STATE: Gelderland
(E) COUNTRY: The Netherlands
(F) POSTAL CODE (ZIP): 6700 AA
(G) TELEPHONE: 31.317477000
(H) TELEFAX: 31.317418094

(ii) TITLE OF INVENTION: Process to collect metabolites from modified
nectar by insects

(iii) NUMBER OF SEQUENCES: 10

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: PCT/NL97/00424

(2) INFORMATION FOR SEQ ID NO: 1:

(i) SEQUENCE CHARACTERISTICS:
(A) LENGTH: 265 amino acids
(B) TYPE: amino acid
(C) STRANDEDNESS:
(D) TOPOLOGY: linear

(ii) MOLECULE TYPE: protein

(iii) HYPOTHETICAL: NO

(iv) ANTI-SENSE: NO

(vi) ORIGINAL SOURCE:
(A) ORGANISM: Petunia hybrida
(B) STRAIN: W115
(F) TISSUE TYPE: Nectar gland

(vii) IMMEDIATE SOURCE:
(B) CLONE: NEC1 amino acid sequence

(xi) SEQUENCE DESCRIPTION: SEQ ID NO: 1:

Met Ala Gln Leu Arg Ala Asp Asp Leu Ser Phe Ile Phe Gly Leu Leu
1               5                   10                  15

Gly Asn Ile Val Ser Phe Met Val Phe Leu Ala Pro Val Pro Thr Phe
            20                  25                  30

Tyr Lys Ile Tyr Lys Arg Lys Ser Ser Glu Gly Tyr Gln Ala Ile Pro
        35                  40                  45

Tyr Met Val Ala Leu Phe Ser Ala Gly Leu Leu Leu Tyr Tyr Ala Tyr
    50                  55                  60

Leu Arg Lys Asn Ala Tyr Leu Ile Val Ser Ile Asn Gly Phe Gly Cys
65                  70                  75                  80

Ala Ile Glu Leu Thr Tyr Ile Ser Leu Phe Leu Phe Tyr Ala Pro Arg
                85                  90                  95

Lys Ser Lys Ile Phe Thr Gly Trp Leu Met Leu Leu Glu Leu Gly Ala
            100                 105                 110

Leu Gly Met Val Met Pro Ile Thr Tyr Leu Leu Ala Glu Gly Ser His
        115                 120                 125

Arg Val Met Ile Val Gly Trp Ile Cys Ala Ala Ile Asn Val Ala Val
    130                 135                 140

Phe Ala Ala Pro Leu Ser Ile Met Arg Gln Val Ile Lys Thr Lys Ser
145                 150                 155                 160

Val Glu Phe Met Pro Phe Thr Leu Ser Leu Phe Leu Thr Leu Cys Ala
                165                 170                 175

Thr Met Trp Phe Phe Tyr Gly Phe Phe Lys Lys Asp Phe Tyr Ile Ala
            180                 185                 190

Phe Pro Asn Ile Leu Gly Phe Leu Phe Gly Ile Val Gln Met Leu Leu
        195                 200                 205

Tyr Phe Val Tyr Lys Asp Ser Lys Arg Ile Asp Asp Glu Lys Ser Asp
    210                 215                 220

Pro Val Arg Glu Ala Thr Lys Ser Lys Glu Gly Val Glu Ile Ile Ile
225                 230                 235                 240

Asn Ile Glu Asp Asp Asn Ser Asp Asn Ala Leu Gln Ser Met Glu Lys
                245                 250                 255

Asp Phe Ser Arg Leu Arg Thr Ser Lys
            260                 265

(2) INFORMATION FOR SEQ ID NO: 2:

    (i) SEQUENCE CHARACTERISTICS:
        (A) LENGTH: 222 amino acids
        (B) TYPE: amino acid
        (C) STRANDEDNESS:
        (D) TOPOLOGY: linear

    (ii) MOLECULE TYPE: protein

    (vi) ORIGINAL SOURCE:
        (A) ORGANISM: Petunia hybrida
        (B) STRAIN: W115
        (C) INDIVIDUAL ISOLATE: FBP15 amino acid sequence
        (F) TISSUE TYPE: Nectar gland
        (G) CELL TYPE: Secretory cell

    (vii) IMMEDIATE SOURCE:
        (A) LIBRARY: cDNA library from nectaries Petunia hybrida
            W115
        (B) CLONE: SEQ2

    (xi) SEQUENCE DESCRIPTION: SEQ ID NO: 2:

Met Gly Arg Gly Lys Ile Glu Ile Lys Arg Ile Glu Asn Asn Arg Thr
1               5                   10                  15

Asn Arg Gln Val Thr Phe Cys Lys Arg Arg Asn Gly Leu Leu Lys Lys
            20                  25                  30

Ala Tyr Glu Leu Ser Val Leu Cys Asp Ala Glu Val Ala Leu Ile Val
        35                  40                  45

Phe Ser Ser Arg Gly Arg Leu Tyr Glu Tyr Ala Asn Asn Ser Val Lys
    50                  55                  60

Ala Thr Ile Asp Arg Tyr Lys Lys Ala Ser Ser Asp Ser Ser Asn Thr
65                  70                  75                  80

Gly Ser Thr Ser Glu Ala Asn Thr Gln Phe Tyr Gln Gln Glu Ala Ala
                85                  90                  95

Lys Leu Arg Val Gln Ile Gly Asn Leu Gln Asn Ser Asn Arg Asn Met
            100                 105                 110

Leu Gly Glu Ser Leu Ser Ser Leu Thr Ala Lys Asp Leu Lys Gly Leu
        115                 120                 125

Glu Thr Lys Leu Glu Lys Gly Ile Ser Arg Ile Arg Ser Lys Lys Asn
    130                 135                 140

Glu Leu Leu Phe Ala Glu Ile Glu Tyr Met Arg Lys Arg Glu Ile Asp
145                 150                 155                 160

Leu His Asn Asn Asn Gln Met Leu Arg Ala Lys Ile Ala Glu Ser Glu
                165                 170                 175

Arg Asn Val Asn Met Met Gly Gly Glu Phe Glu Leu Met Gln Ser His
            180                 185                 190

Pro Tyr Asp Pro Arg Asp Phe Phe Gln Val Asn Gly Leu Gln His Asn
        195                 200                 205

His Gln Tyr Pro Arg Gln Asp Asn Met Ala Leu Gln Leu Val
    210                 215                 220

(2) INFORMATION FOR SEQ ID NO: 3:

    (i) SEQUENCE CHARACTERISTICS:
        (A) LENGTH: 18 amino acids
        (B) TYPE: amino acid
        (C) STRANDEDNESS:
        (D) TOPOLOGY: linear

    (ii) MOLECULE TYPE: protein

    (iii) HYPOTHETICAL: NO

    (iv) ANTI-SENSE: NO

    (v) FRAGMENT TYPE: N-terminal

    (vi) ORIGINAL SOURCE:
        (A) ORGANISM: Calluna vulgaris
        (F) TISSUE TYPE: flower

    (vii) IMMEDIATE SOURCE:
        (B) CLONE: Calluna vulgaris signal peptide

    (xi) SEQUENCE DESCRIPTION: SEQ ID NO: 3:

Met Phe Leu Pro Ile Leu Phe Thr Ile Ser Leu Leu Phe Ser Ser Ser
1               5                   10                  15

His Ala

(2) INFORMATION FOR SEQ ID NO: 4:

(i) SEQUENCE CHARACTERISTICS:
(A) LENGTH: 1205 base pairs
(B) TYPE: nucleic acid
(C) STRANDEDNESS: double
(D) TOPOLOGY: linear

(ii) MOLECULE TYPE: cDNA

(iii) HYPOTHETICAL: NO

(iv) ANTI-SENSE: NO

(vi) ORIGINAL SOURCE:
(A) ORGANISM: Petunia hybrida
(B) STRAIN: W115
(F) TISSUE TYPE: Nectar gland

(vii) IMMEDIATE SOURCE:
(B) CLONE: NEC1 nucleotide sequence

(ix) FEATURE:
(A) NAME/KEY: CDS
(B) LOCATION:79..873

(xi) SEQUENCE DESCRIPTION: SEQ ID NO: 4:

TCGAGCGGCC GCCCGGGCAG GTATTCAACA AGAGTATTCA CCACTTGAAC TCAAAAGGGG 60

CTTCACTAAA AAAAAATC ATG GCG CAA TTA CGT GCT GAT GAC TTG TCT TTC 111
Met Ala Gln Leu Arg Ala Asp Asp Leu Ser Phe
1               5                   10

ATA TTT GGC CTT CTT GGT AAT ATT GTA TCA TTC ATG GTC TTC CTA GCA 159
Ile Phe Gly Leu Leu Gly Asn Ile Val Ser Phe Met Val Phe Leu Ala
            15                  20                  25

CCC GTG CCA ACA TTT TAC AAA ATA TAT AAA AGG AAA TCA TCA GAA GGA 207
Pro Val Pro Thr Phe Tyr Lys Ile Tyr Lys Arg Lys Ser Ser Glu Gly
        30                  35                  40

TAT CAA GCA ATA CCA TAT ATG GTA GCA CTG TTC AGC GCC GGA CTA TTG 255
Tyr Gln Ala Ile Pro Tyr Met Val Ala Leu Phe Ser Ala Gly Leu Leu
    45                  50                  55

CTA TAT TAT GCT TAT CTC AGG AAG AAT GCC TAT CTT ATC GTC AGC ATT 303
Leu Tyr Tyr Ala Tyr Leu Arg Lys Asn Ala Tyr Leu Ile Val Ser Ile
60                  65                  70                  75

AAT GGC TTT GGA TGT GCC ATT GAA TTA ACA TAT ATC TCT CTG TTT CTC 351
Asn Gly Phe Gly Cys Ala Ile Glu Leu Thr Tyr Ile Ser Leu Phe Leu
                80                  85                  90

TTT TAC GCG CCC AGA AAG TCT AAG ATT TTC ACA GGG TGG CTG ATG CTC 399
Phe Tyr Ala Pro Arg Lys Ser Lys Ile Phe Thr Gly Trp Leu Met Leu
            95                  100                 105

TTA GAA TTG GGA GCC CTA GGA ATG GTG ATG CCA ATT ACT TAT TTA TTA 447
Leu Glu Leu Gly Ala Leu Gly Met Val Met Pro Ile Thr Tyr Leu Leu
        110                 115                 120

GCA GAA GGC TCA CAT AGA GTG ATG ATA GTG GGA TGG ATT TGT GCA GCT 495
Ala Glu Gly Ser His Arg Val Met Ile Val Gly Trp Ile Cys Ala Ala
    125                 130                 135

ATC AAT GTT GCT GTC TTT GCT GCT CCT TTA AGC ATC ATG AGG CAA GTA 543
Ile Asn Val Ala Val Phe Ala Ala Pro Leu Ser Ile Met Arg Gln Val
140                 145                 150                 155

ATA AAA ACA AAG AGT GTA GAG TTC ATG CCC TTC ACT TTA TCT TTG TTC 591
Ile Lys Thr Lys Ser Val Glu Phe Met Pro Phe Thr Leu Ser Leu Phe
                160                 165                 170

CTC ACT CTC TGT GCC ACT ATG TGG TTT TTC TAT GGG TTT TTC AAG AAG 639
Leu Thr Leu Cys Ala Thr Met Trp Phe Phe Tyr Gly Phe Phe Lys Lys
            175                 180                 185

GAC TTT TAC ATT GCG TTT CCA AAT ATA CTG GGC TTT CTA TTC GGA ATC 687
Asp Phe Tyr Ile Ala Phe Pro Asn Ile Leu Gly Phe Leu Phe Gly Ile
        190                 195                 200

GTT CAA ATG CTA TTA TAT TTT GTT TAC AAG GAT TCA AAG AGA ATA GAT 735
Val Gln Met Leu Leu Tyr Phe Val Tyr Lys Asp Ser Lys Arg Ile Asp
    205                 210                 215

GAT GAA AAA TCT GAT CCT GTT CGA GAA GCT ACA AAA TCA AAA GAA GGT 783
Asp Glu Lys Ser Asp Pro Val Arg Glu Ala Thr Lys Ser Lys Glu Gly
220                 225                 230                 235

GTA GAA ATC ATT ATC AAC ATT GAA GAT GAT AAT TCT GAT AAC GCA TTG 831
Val Glu Ile Ile Ile Asn Ile Glu Asp Asp Asn Ser Asp Asn Ala Leu
                240                 245                 250

CAG TCC ATG GAG AAG GAT TTT TCC AGA CTG CGG ACA TCA AAA 873
Gln Ser Met Glu Lys Asp Phe Ser Arg Leu Arg Thr Ser Lys
            255                 260                 265

TAAGCAAGAA GATGATCAAA AAATGACAAA GCTAAGGAGT TTGAAGTAAG GCAAGGAACT 933

TGACACTGAA TATCTAAGCT AATTAGCAAG ACTTTAGCAG CTTGTAATAT TTAGTGTTTG 993

TGAGGTGTTA CCTTATAATT AGCTTGTAGC ATAGCCTTCC CACTAATAAT TCTGCTTAGC 1053

GAATCTTATA TATGGGAAAT ACTTACACTA GTATGCATCT TCTATATACA TGTTTGGCAC 1113

TTGACTATAC ATAGAAAAAT TAACAAGCAT TTCTCACCTC AATTTGTCAC TTACTTATAA 1173

GTAGCTGAAT AATATAATGC AATTTTCACC CC 1205

(2) INFORMATION FOR SEQ ID NO: 5:

(i) SEQUENCE CHARACTERISTICS:
(A) LENGTH: 265 amino acids
(B) TYPE: amino acid
(D) TOPOLOGY: linear

(ii) MOLECULE TYPE: protein
(xi) SEQUENCE DESCRIPTION: SEQ ID NO: 5:

Met Ala Gln Leu Arg Ala Asp Asp Leu Ser Phe Ile Phe Gly Leu Leu
1               5                   10                  15

Gly Asn Ile Val Ser Phe Met Val Phe Leu Ala Pro Val Pro Thr Phe
            20                  25                  30

Tyr Lys Ile Tyr Lys Arg Lys Ser Ser Glu Gly Tyr Gln Ala Ile Pro
        35                  40                  45

Tyr Met Val Ala Leu Phe Ser Ala Gly Leu Leu Leu Tyr Tyr Ala Tyr
    50                  55                  60

Leu Arg Lys Asn Ala Tyr Leu Ile Val Ser Ile Asn Gly Phe Gly Cys
65                  70                  75                  80

Ala Ile Glu Leu Thr Tyr Ile Ser Leu Phe Leu Phe Tyr Ala Pro Arg
                85                  90                  95

Lys Ser Lys Ile Phe Thr Gly Trp Leu Met Leu Leu Glu Leu Gly Ala
            100                 105                 110

Leu Gly Met Val Met Pro Ile Thr Tyr Leu Leu Ala Glu Gly Ser His
        115                 120                 125

Arg Val Met Ile Val Gly Trp Ile Cys Ala Ala Ile Asn Val Ala Val
    130                 135                 140

Phe Ala Ala Pro Leu Ser Ile Met Arg Gln Val Ile Lys Thr Lys Ser
145                 150                 155                 160

Val Glu Phe Met Pro Phe Thr Leu Ser Leu Phe Leu Thr Leu Cys Ala
                165                 170                 175

Thr Met Trp Phe Phe Tyr Gly Phe Phe Lys Lys Asp Phe Tyr Ile Ala
            180                 185                 190

Phe Pro Asn Ile Leu Gly Phe Leu Phe Gly Ile Val Gln Met Leu Leu
        195                 200                 205

Tyr Phe Val Tyr Lys Asp Ser Lys Arg Ile Asp Asp Glu Lys Ser Asp
    210                 215                 220

Pro Val Arg Glu Ala Thr Lys Ser Lys Glu Gly Val Glu Ile Ile Ile
225                 230                 235                 240

Asn Ile Glu Asp Asp Asn Ser Asp Asn Ala Leu Gln Ser Met Glu Lys
                245                 250                 255

Asp Phe Ser Arg Leu Arg Thr Ser Lys
            260                 265

(2) INFORMATION FOR SEQ ID NO: 6:

     (i) SEQUENCE CHARACTERISTICS:
          (A) LENGTH: 1157 base pairs
          (B) TYPE: nucleic acid
          (C) STRANDEDNESS: double
          (D) TOPOLOGY: linear

    (ii) MOLECULE TYPE: cDNA

   (iii) HYPOTHETICAL: NO

    (iv) ANTI-SENSE: NO

    (vi) ORIGINAL SOURCE:
          (A) ORGANISM: Petunia hybrida
          (B) STRAIN: W115
          (F) TISSUE TYPE: Nectar gland

   (vii) IMMEDIATE SOURCE:
          (A) LIBRARY: cDNA library of nectaries from Petunia
               hybrida flowers
          (B) CLONE: FBP15 nucleotide sequence

    (ix) FEATURE:
          (A) NAME/KEY: CDS
          (B) LOCATION:179..841

    (xi) SEQUENCE DESCRIPTION: SEQ ID NO: 6:

TCTGAATACA AGCTGTGTGT GTAGAGAGAT TTCATAAAGA CAGCAAACAT CCCTTCTTTT        60

TGTTCTGTTT TAAAAGTTCC CTTCTTCAAC CAGCTCTTTT CCTCATCAGG GTAAGTTGCA 120

AATAAAGGGG ATGTTCCAGA ATCAAGAAGA GAAGATGTCA GACTCGCCTC AGAGGAAG 178

ATG GGA AGA GGA AAG ATT GAG ATT AAG AGG ATT GAA AAT ACA ACA AAT 226
Met Gly Arg Gly Lys Ile Glu Ile Lys Arg Ile Glu Asn Thr Thr Asn
1               5                   10                  15

CGT CAA GTC ACT TTC TGT AAG AGA AGA AAT GGG TTG CTT AAA AAA GCT 274
Arg Gln Val Thr Phe Cys Lys Arg Arg Asn Gly Leu Leu Lys Lys Ala
            20                  25                  30

TAT GAA CTT TCT GTT CTT TGT GAT GCT GAA GTT GCT CTC ATC GTT TTC 322
Tyr Glu Leu Ser Val Leu Cys Asp Ala Glu Val Ala Leu Ile Val Phe
        35                  40                  45

TCA AGC CGT GGC CGC CTC TAT GAA TAT GCT AAC AAC AGT GTG AAG GCA 370
Ser Ser Arg Gly Arg Leu Tyr Glu Tyr Ala Asn Asn Ser Val Lys Ala
    50                  55                  60

ACA ATT GAT AGA TAT AAG AAA GCA TCC TCA GAT TCC TCC AAC ACT GGA 418
Thr Ile Asp Arg Tyr Lys Lys Ala Ser Ser Asp Ser Ser Asn Thr Gly
65                  70                  75                  80

TCT ACT TCT GAA GCT AAC ACT CAG TTT TAT CAA CAA GAA GCT GCC AAA 466
Ser Thr Ser Glu Ala Asn Thr Gln Phe Tyr Gln Gln Glu Ala Ala Lys
                85                  90                  95

CTC CGA GTT CAG ATT GGT AAC TTA CAG AAC TCA AAC AGG AAC ATG CTA 514
Leu Arg Val Gln Ile Gly Asn Leu Gln Asn Ser Asn Arg Asn Met Leu
            100                 105                 110

GGC GAG TCT CTA AGT TCT CTG ACT GCA AAA GAT CTG AAA GGC CTG GAG 562
Gly Glu Ser Leu Ser Ser Leu Thr Ala Lys Asp Leu Lys Gly Leu Glu
        115                 120                 125

ACC AAA CTT GAG AAA GGA ATT AGT AGA ATT AGG TCC AAA AAG AAT GAA 610
Thr Lys Leu Glu Lys Gly Ile Ser Arg Ile Arg Ser Lys Lys Asn Glu
    130                 135                 140

CTC CTG TTT GCT GAG ATT GAG TAT ATG CGA AAA AGG GAA ATT GAT TTG 658
Leu Leu Phe Ala Glu Ile Glu Tyr Met Arg Lys Arg Glu Ile Asp Leu
145                 150                 155                 160

CAC AAC AAC AAT CAG ATG CTT CGG GCA AAG ATA GCT GAG AGT GAA AGA 706
His Asn Asn Asn Gln Met Leu Arg Ala Lys Ile Ala Glu Ser Glu Arg
                165                 170                 175

AAT GTG AAC ATG ATG GGA GGA GAA TTT GAG CTG ATG CAA TCT CAT CCG 754
Asn Val Asn Met Met Gly Gly Glu Phe Glu Leu Met Gln Ser His Pro
            180                 185                 190

TAC GAT CCA AGA GAC TTC TTC CAA GTG AAC GGC TTA CAG CAT AAT CAT 802
Tyr Asp Pro Arg Asp Phe Phe Gln Val Asn Gly Leu Gln His Asn His
        195                 200                 205

CAA TAT CCA CGC CAA GAC AAC ATG GCT CTT CAA TTA GTA TAAGTTTATA 851
Gln Tyr Pro Arg Gln Asp Asn Met Ala Leu Gln Leu Val
    210                 215                 220

ATAAAATGCA TGGTTTGAAG CACTCTGATT GTGGTGGATT TGGATTATGT ATAAGGGAGT 911

GCAGGCCATT TGCCAATTAT TGAAAGGTAC TCAAACAGGA AGTTGAAGAA GTTCATCATC 971

TCTCTCATCT ATATGTCTTA ACAAAAGTCT TAGCTTATGG ACTCTAAAAC AAAGACTTAA 1031

TTTAACATAT AAATATAATT GTGTAATGCT GTTGTATTGT ATGGTATGTA TCCAAAAACA 1091

TTAATAACCT ATCTTTTTCT TCAAATTATG TCTCCTTTGA TACAAACTAC TAACATATTT 1151

TCTTAT                                                                    1157

(2) INFORMATION FOR SEQ ID NO: 7:

        (i) SEQUENCE CHARACTERISTICS:
            (A) LENGTH: 221 amino acids
            (B) TYPE: amino acid
            (D) TOPOLOGY: linear

    (ii) MOLECULE TYPE: protein
    (xi) SEQUENCE DESCRIPTION: SEQ ID NO: 7:

Met Gly Arg Gly Lys Ile Glu Ile Lys Arg Ile Glu Asn Thr Thr Asn
 1               5                  10                  15

Arg Gln Val Thr Phe Cys Lys Arg Arg Asn Gly Leu Leu Lys Lys Ala
            20                  25                  30

Tyr Glu Leu Ser Val Leu Cys Asp Ala Glu Val Ala Leu Ile Val Phe
        35                  40                  45

Ser Ser Arg Gly Arg Leu Tyr Glu Tyr Ala Asn Asn Ser Val Lys Ala
    50                  55                  60

Thr Ile Asp Arg Tyr Lys Lys Ala Ser Ser Asp Ser Ser Asn Thr Gly
65                  70                  75                  80

Ser Thr Ser Glu Ala Asn Thr Gln Phe Tyr Gln Gln Glu Ala Ala Lys
                85                  90                  95

Leu Arg Val Gln Ile Gly Asn Leu Gln Asn Ser Asn Arg Asn Met Leu
            100                 105                 110

Gly Glu Ser Leu Ser Ser Leu Thr Ala Lys Asp Leu Lys Gly Leu Glu
        115                 120                 125

Thr Lys Leu Glu Lys Gly Ile Ser Arg Ile Arg Ser Lys Lys Asn Glu
    130                 135                 140

Leu Leu Phe Ala Glu Ile Glu Tyr Met Arg Lys Arg Glu Ile Asp Leu
145                 150                 155                 160

His Asn Asn Asn Gln Met Leu Arg Ala Lys Ile Ala Glu Ser Glu Arg
                165                 170                 175

Asn Val Asn Met Met Gly Gly Glu Phe Glu Leu Met Gln Ser His Pro
            180                 185                 190

Tyr Asp Pro Arg Asp Phe Phe Gln Val Asn Gly Leu Gln His Asn His
        195                 200                 205

Gln Tyr Pro Arg Gln Asp Asn Met Ala Leu Gln Leu Val
    210                 215                 220

(2) INFORMATION FOR SEQ ID NO: 8:

        (i) SEQUENCE CHARACTERISTICS:
            (A) LENGTH: 54 base pairs
            (B) TYPE: nucleic acid
            (C) STRANDEDNESS: double
            (D) TOPOLOGY: linear

    (ii) MOLECULE TYPE: DNA (genomic)

    (iii) HYPOTHETICAL: NO

    (iv) ANTI-SENSE: NO

(vi) ORIGINAL SOURCE:
(A) ORGANISM: Calluna vulgaris

(vii) IMMEDIATE SOURCE:
(B) CLONE: Signal peptide Calluna vulgaris, nucleotide
sequence

(ix) FEATURE:
(A) NAME/KEY: CDS
(B) LOCATION:1..54

(xi) SEQUENCE DESCRIPTION: SEQ ID NO: 8:

ATG TTT CTT CCA ATT CTC TTC ACC ATT TCC CTC CTC TTC TCC TCC TCC 48
Met Phe Leu Pro Ile Leu Phe Thr Ile Ser Leu Leu Phe Ser Ser Ser
1               5                   10                  15

CAT GCT 54
His Ala

(2) INFORMATION FOR SEQ ID NO: 9:

(i) SEQUENCE CHARACTERISTICS:
(A) LENGTH: 18 amino acids
(B) TYPE: amino acid
(D) TOPOLOGY: linear

(ii) MOLECULE TYPE: protein
(xi) SEQUENCE DESCRIPTION: SEQ ID NO: 9:

Met Phe Leu Pro Ile Leu Phe Thr Ile Ser Leu Leu Phe Ser Ser Ser
1               5                   10                  15

His Ala

(2) INFORMATION FOR SEQ ID NO: 10:

(i) SEQUENCE CHARACTERISTICS:
(A) LENGTH: 2141 base pairs
(B) TYPE: nucleic acid
(C) STRANDEDNESS: double
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Petunia hybrida
(B) STRAIN: W115

(vii) IMMEDIATE SOURCE:
(B) CLONE: NEC1 promoter

(xi) SEQUENCE DESCRIPTION: SEQ ID NO: 10:

CCTAGGAGAA ATCAAGCCTA CTCTTAAGAT GGATGACTCA CTTGCCCCGA TGGTAAGGTG 60

AAGGATCTGT TGATTAGAGT TGGGAAGTTC ATGTTCTCTG CTGATTTTAT TATTCTAGAC 120

TATGAAGAGG ACCAAGAAGC TCCAATAATT TTGGGAAGAG CATTCTTAAT CACATCGATG 180

GCAATTATTG ACATGGAACT TGGGGAGATG ACTGTGAGAG CGCATGGAGA AAAGGTTACT 240
TTCAAGGTTT ATAATAAAAA GGATCATATG GCTAAGTTTG AAGAGTGTTC TTTGATAGAA 300
TGTGTCAGAC GAGAACATGA AAGTAAACCG AAAGAGGTGT TTGAGCGGAA TGTAGAACAA 360
AGTGACCACG GCACAATAAT TGACAAGTTG AAGGAAAATT CACCTAAAGG AAGGAAGAAG 420
ACAAAAGTTC GTCGTAACAA GAGGAGACGT AAATGCTGGA AGTGAGCTTA AAGGTGTTGT 480
CGTACTACGA CGTTAACTAA GGCGCTTGTC GGGAGGCAAC CCTAGCTTTG TATGTAAATG 540
TAAAAGTAAA AAATATATAT ATAGAAAAAG GAAAATACAA AAAGAGTCGT GCCGCGACGT 600
TAAATCAAGC GCTTGTTGGA AGGCAACCCA ATTTTTATTG TTTTAGTTGT TTTACTTATT 660
TAGTATTACG TAGTTTCTTG TTGTTTTTGT AGGGCTCGGG ACTTTCGGAA GGTGAGGTAA 720
TTTCAAGGCA TCGCGGTGTG TATTGCAGCG AGGTAAGTGT AAGAGTTGAG TTGGAAGCGT 780
TTGGCCAAGT GTTGCACCGT GAGAGGCTTT CAACCTGTTG CGACACGTGA AAAATTAAGA 840
GCCAGATCTG CTACATTAGC ACTGAAGCAT CGCTTGGCCA ATAGCTTGGA ATGGAAGCAA 900
GAATTCAAAC CAAAATCAGA AACGCCACAA GAGATGTGTC GCACACTGCA AAGCTTTGTG 960
CAAACTAGTG AACGCAGAAA TAGAAATGCT ACAGCCCATG CGTCGCTTGG CTTATGGCAG 1020
GCAGCAAAAA TTCAGCAGCA AAACAGAAAC GCTGCGAGAA ACGCGTCGCA TACGCCATAG 1080
CTTTGTGTCA AACAGAACGT CCAGAAATTG AAAAGCTATA AGCCTGCGTC GCTTGGCTCA 1140
TGGCGTGCAG ACTAGAAAAG CTCTAGCAGA TGCGTCGCGT ATTGTATAGC TTGGTGTGAA 1200
ACAGAAAGTT CGAAACTTGG AAAACGATAA CCCAGCGTCG CCTCTTCAAC CGCGTCCAGG 1260
TAAGTTCAAG ATTCTTACGG GTTGACCCAT TAACCCATTG ATCGGCTGAT TATAAACAAT 1320
AAAACATCAC CTTCAACTAT CACATGATTT CATAAGTTTG ACCTAGGATA TTTTATATAT 1380
ATATATATAT ATATACACAC ACACACCATT TCCAGCGATC TTACCTCATT TTTATTCAAA 1440
CCATTTTTCT GCTTCAAAAG TTTAAATTAT TAATATGATA AGTCATCCAT AGTCAAACAA 1500
GATTTTCTAT ACTATTTTGT CCCTTGTAAT TTTAAAAAAA AAATGAGCGA TGGTAAGATA 1560
AACATTGTTT GCAAGTGTAC AATTTTAGTA TATGCAAACC AACGCTTCTT CTTCCAACTA 1620
TCACCTAAAA CTACATCATT TATGGCGGGC GGACTAGACG TAGCCAAATA TAAAAACGCA 1680
ATGGCCATTC AGTTCATGTC ATTTTTATAT CCTTCATCCA ATAATATTAC TCAAAATTGA 1740
TGTACAGTTT GGTCTCTGAT GTGCACTTTA CTATACGTAA TACGGAATTT ACATTATAAT 1800
TAAAGAGAAC TGTTCCACTA AATTTTAATG ATTTAATTAA TTTAACTCGG TTACTTGTAT 1860
TATTATTATT GCTGTATTTG TTTGTCATTT GAATTTGGCA CCGCAGATTT TTGTATGCAA 1920
TTAACCCTCA TATATCTTTT GGCCAAATAA AGAAAAAGTC TGCATATTTC TTGCCAAACA 1980
TTTATCATAC TTTACCGAAT TCTTGTTTTT TGTTTCTCTG TTGTTGTTCT CCACTATAAA 2040
TAACATTTGC AGTGAGTAAA GTTTCTTCAG GTCTCTTTTG TAGATTCAAC AAGAGTATTC 2100
AGCACTTGAA CTCAAAAGGG GCTTCACTAA AAAAAATCAT G 2141